# EUROPEAN PATENT APPLICATION

(11) **EP 4 851 179 A1**
(43) Date of publication of application: **09.12.2026**
(21) Application number: 25748666.2
(22) Date of filing: 29.01.2025
(51) Int. Cl.: C07K 4/00, C07K 7/08, C07K 16/00, C07K 19/00, C12N 15/11, C12N 15/13, C12N 15/62, C12N 15/63

(54) **POLYPEPTIDE AND USE THEREOF**

(30) Priority: 29.01.2024 JP 2024011182
(71) Applicant: Tanabe Pharma Corporation, Osaka-shi, Osaka 541-8505 (JP)
(72) Inventor: WADA Koichi, Osaka-shi, Osaka 541-8505 (JP)
(74) Representative: J A Kemp LLP
(86) International application number: PCT/JP2025/002777
(87) International publication number: WO 2025/164663

(57) **Abstract**

A polypeptide for improving binding of an antigen-binding polypeptide to an antigen within cells has 3 or more and 20 or less amino acids, wherein more than 57.0% and less than 100.0% of the amino acids are acidic amino acids. A fusion polypeptide includes the polypeptide and an antigen-binding polypeptide.

## Description

### Technical Field

The present disclosure relates to a polypeptide and use thereof. The present application claims priority based on Japanese Patent Application No. 2024-011182, filed in Japan on January 29, 2024, the contents of which are incorporated herein by reference.

### Technical Background

It is known that when attempting to obtain antibodies selective for intracellular proteins by phage antibody display or animal immunization, and then express those antibodies intracellularly to act on a target antigen protein, in many cases the expressed antibodies themselves aggregate, preventing an intended action from being sufficiently exerted.

Further, it has been suggested that when antibodies aggregate intracellularly, not only is an intended action not exerted, but there is also a possibility of inducing cytotoxicity, raising concerns about increased safety risks when used for therapeutic applications (see, for example, Non-Patent Document 1). Therefore, in order to obtain antibodies that act on intracellular target antigen proteins, antibody engineering for enabling intracellular functioning is required; however, this is generally not easy to carry out.

In recent years, a peptide fusion technology capable of suppressing intracellular antibody self-aggregation has been reported, making it possible to obtain intracellularly stable antibodies (see, for example, Patent Document 1).

### Related Art

### Patent Document

Patent Document 1: International Publication No. 2019/004213.

### Non-Patent Document

Non-Patent Document 1: H. Kabayama et al., "An ultra-stable cytoplasmic antibody engineered for in vivo applications," Nature Communications, Vol. 11, No. 336, pp. 1-20, 2020.

### Summary of the Invention

### Problems to Be Solved by the Invention

However, studies by the inventor have revealed that when expressing intracellularly an antibody to which a peptide described in Patent Document 1 or the like has been attached, an amount of antibody expressed within cells may be significantly reduced depending on the type of peptide to be fused, and there is a risk that an intended binding effect on a target antigen protein within cells may not always be sufficiently exerted. In order to for an antigen-binding polypeptide such as an intracellular antibody to be used for medical applications, since a level of intracellular expression is highly likely to affect a level of therapeutic efficacy, there is a need for such a polypeptide to be expressed more stably and to maintain a superior binding function to a target antigen protein within cells, and development of a technology capable of providing such effects is desired.

The present disclosure has been made in view of the above circumstances, and provides a polypeptide capable of improving the binding of an antigen-binding polypeptide to an antigen within cells.

### Means for Solving the Problems

That is, the present disclosure includes the following aspect.

A polypeptide according to one aspect of the present disclosure is a polypeptide for improving binding of an antigen-binding polypeptide to an antigen within cells. The polypeptide contains 3 or more and 20 or less amino acids, and more than 57.0% and less than 100.0% of the amino acids are acidic amino acids.

### Effects of Invention

According to the polypeptide of the above aspect, binding of an antigen-binding polypeptide to an antigen within cells can be improved.

### Brief Description of Drawing

[Fig. 1] A figure showing results of Experimental Example 1.
[Fig. 2] A figure showing results of Experimental Example 2.
[Fig. 3] A figure showing results of Experimental Example 3.
[Fig. 4] A figure showing results of Experimental Example 4.
[Fig. 5] A figure showing results of Experimental Example 5.
[Fig. 6] A figure showing results of Experimental Example 6.
[Fig. 7] A figure showing results of Experimental Example 7.
[Fig. 8] A figure showing results of Experimental Example 8.
[Fig. 9] A figure showing results of Experimental Example 9.
[Fig. 10] A figure showing results of Experimental Example 10.
[Fig. 11] A figure showing results of Experimental Example 11.
[Fig. 12] A figure showing results of Experimental Example 12.

### Mode for Carrying Out the Invention

### <Definition of Terms>

As used herein, "polypeptide" is also referred to as "peptide" or "protein." "Polypeptide" means a compound in which two or more amino acids are linked by peptide bonds. A polypeptide may contain, for example, a structure modified with a sugar or the like. The amino acids in a polypeptide may be naturally occurring amino acids, or may be known analogs of naturally occurring amino acids that can function in a manner similar to naturally occurring amino acids. The amino acids may be L-amino acids or D-amino acids.

As used herein, "acidic amino acid" means an amino acid having an isoelectric point of 3.9 or less. Acidic amino acids may be naturally occurring amino acids or may be known analogs of naturally occurring amino acids. Examples of naturally occurring acidic amino acids include aspartic acid (D), glutamic acid (E), and the like.

As used herein, "basic amino acid" means an amino acid having an isoelectric point of 7.4 or above. Basic amino acids may be naturally occurring amino acids or may be known analogs of naturally occurring amino acids. Examples of naturally occurring basic amino acids include histidine (H), lysine (K), arginine (R), and the like.

As used herein, "neutral amino acid" means an amino acid other than the above acidic amino acids and the above basic amino acids. That is, a neutral amino acid means an amino acid having an isoelectric point of more than 3.9 and less than 7.4. Neutral amino acids may be naturally occurring amino acids or may be known analogs of naturally occurring amino acids. Examples of naturally occurring neutral amino acids include alanine (A), asparagine (N), cysteine (C), phenylalanine (F), glycine (G), isoleucine (I), leucine (L), methionine (M), proline (P), glutamine (Q), serine (S), threonine (T), valine (V), tryptophan (W), tyrosine (Y), and the like.

As used herein, "antigen" means any molecule to which an antigen-binding polypeptide can bind. Specific examples of antigens include proteins, peptides, polysaccharides, glycoproteins, glycolipids, nucleic acids, portions thereof, combinations thereof, and the like. An antigen may be synthetically produced or may be derived from a biological sample. Examples of such biological samples include, but are not limited to, tissue samples, tumor samples, cells, or other biological components, organisms, subunits of proteins/antigens, liquids containing killed or inactivated whole cells or cell lysates, and the like.

As used herein, "antigen-binding polypeptide" means a polypeptide that specifically binds to an antigen (target molecule). An antigen-binding polypeptide has all or a portion of a region having antigen-binding ability. Examples of antigen-binding polypeptides include, but are not limited to, antibodies and antigen-binding fragments thereof, peptide aptamers, and the like.

As used herein, "antibody" may be a monoclonal antibody or a polyclonal antibody. A monoclonal antibody may be a naturally occurring antibody or an antibody produced using recombinant DNA technology. Naturally occurring antibodies are not particularly limited, but can be derived from biological species including humans, mice, rats, monkeys, goats, rabbits, camels, llamas, cattle, chickens, and the like. Examples of antibodies produced using recombinant DNA technology include, but are not particularly limited to, synthetic antibodies, recombinant antibodies, mutagenized antibodies, and the like, produced based on naturally occurring antibodies. Also included are antibodies obtained by modifying antibodies already produced using recombinant DNA technology in a manner similar to the genetic modification of naturally occurring antibodies as described above. Monoclonal antibodies include multispecific antibodies, single-chain antibodies, Fab fragments, Fab' fragments, F(ab') fragments, F(ab')2 fragments, Fv (variable fragment of antibody), single-chain Fv (scFv), dsFv (disulphide stabilized Fv), dAb (single domain antibody), VH, VL, intrabodies, minibodies, nanobodies (variable domain of heavy chain of heavy chain antibody, VHH), vNAR, diabodies, full-length anti-idiotype (anti-Id) antibodies or portions thereof, and the like.

As used herein, "intrabody" means an antibody (intracellular antibody) that specifically binds to an antigen intracellularly.

As used herein, "nucleic acid" is also referred to as "nucleotide" or "polynucleotide." Unless otherwise specified, nucleic acid encompasses both DNA and RNA.

As used herein, "expression vector" means a vector that contains a regulatory sequence and a nucleic acid encoding a protein operably linked to the regulatory sequence, and is capable of transcribing mRNA encoding the protein in a cell or in vitro. An expression vector can contain, for example, an origin of replication for replicating in a cell, and a regulatory sequence and a nucleic acid encoding a protein operably linked to the regulatory sequence. An expression vector can have a selection marker for selecting cells into which the expression vector has been introduced, for example, a drug resistance gene.

Examples of regulatory sequences for expression in mammalian cells include, but are not particularly limited to, promoters for RNA polymerase II, such as cytomegalovirus (CMV) immediate early (IE), HSV thymidine kinase, SV40 early, SV40 late, retroviral LTR, and metallothionein I promoters, and the like.

Examples of promoters for expression in Escherichia coli include lac, trp, lacI, lacZ, T3, T7, gpt, lambda PR, lambda PL, and 3-phosphoglycerate kinase promoters.

Examples of promoters for expression in vitro include, but are not particularly limited to, SP6, T7, and T3 promoters, and the like.

Examples of expression vectors include plasmids, phages, phagemids, cosmids, fosmids, artificial chromosomes, retroviral vectors, lentiviral vectors, measles virus vectors, vaccinia virus vectors, adenoviral vectors, adeno-associated virus vectors, Sendai virus vectors, and the like.

### <Polypeptide for Improving Binding of an Antigen-Binding Polypeptide to an Antigen within Cells>

A polypeptide of the present embodiment is for improving binding of an antigen-binding polypeptide to an antigen within cells. The polypeptide of the present embodiment contains 3 or more and 20 or less amino acids. More than 57.0% and less than 100.0% of the amino acids in the polypeptide of the present embodiment are acidic amino acids.

As shown in Experimental Example 6 described below, the inventors found that in a fusion polypeptide in which a polypeptide described in Patent Document 1 or the like, which is known to enhance intracellular antibody stability, has been attached to the N-terminus of an antigen polypeptide, a level of intracellular expression is remarkably low, and as a result, a function of the antigen-binding polypeptide within cells cannot be sufficiently exerted.

On the other hand, it is known that ribosome stalling is significantly affected when "E" is present at an A-site, "P," "G," or "D" at a P-site, and "E" or "D" at an E-site of ribosome, and that translation by ribosome is destabilized by having a DE/P motif and/or having an amino acid of a small molecular size at the N-terminus (see Reference 1: K Chyzynska et al., "Deep conservation of ribosome stall sites across RNA processing genes," NAR Genomics and Bioinformatics, Vol. 3, Issue 2, 2021, pp. 1-26).

Based on a description in Reference 1, the inventor has hypothesized that a relatively long polypeptide containing 21 or more amino acids and having consecutive acidic amino acids would destabilize ribosomal translation and, as a result, would significantly reduce an expression level of an antigen-binding polypeptide within cells, and thus, while such a polypeptide is favorable from a viewpoint of suppressing intracellular antibody aggregation, it is not suitable from a viewpoint of medical applications.

When an antigen-binding polypeptide such as an intrabody is used for medical applications, since an expression level of the antigen-binding polypeptide within cells affects pharmacological efficacy, there is a need for development of a technology that enables stable expression within cells and improves binding ability to a target antigen.

Based on the above problem and the above hypothesis, as a result of extensive studies, the inventor has found that, by attaching to the N-terminus of an antigen-binding polypeptide a polypeptide containing an amino acid sequence having acidic amino acids (E and D) as major constituents and having a specific structure, binding to a target antigen within cells can be improved without significantly reducing an expression level of the antigen-binding polypeptide within cells, thereby completing the polypeptide of the present embodiment.

By attaching the polypeptide of the present embodiment to the N-terminus of an antigen-binding polypeptide, when a fusion polypeptide of the polypeptide of the present embodiment and the antigen-binding polypeptide is expressed within cells, a translation rate of ribosome is slightly modulated, folding efficiency of the antigen-binding polypeptide is improved, and a proportion of antigen-binding polypeptides maintaining correct folding within cells is increased. As a result, it is presumed that the binding of the antigen-binding polypeptide to an antigen within cells is improved.

The polypeptide of the present embodiment can also be characterized as a polypeptide for modulating a translation rate of an antigen-binding polypeptide within cells and improving folding efficiency of the antigen-binding polypeptide. Alternatively, the polypeptide of the present embodiment can also be characterized as a polypeptide for increasing a proportion of antigen-binding polypeptides maintaining correct folding within cells.

However, a mechanism by which the polypeptide of the present embodiment improves binding of an antigen-binding polypeptide to an antigen within cells is not limited to the above mechanism.

Improvement in binding of an antigen-binding polypeptide to an antigen within cells can be evaluated, for example, using the following methods. That is, first, an expression vector containing a nucleic acid encoding an antigen-binding polypeptide to which the polypeptide of the present embodiment has been attached at its N-terminus, and an expression vector containing only a nucleic acid encoding an antigen-binding polypeptide to which the polypeptide of the present embodiment has not been attached, are prepared, and these expression vectors are each introduced into cells and expressed within the cells. Next, the antigen-binding polypeptide is recovered by immunoprecipitation, and the antigen-binding polypeptide and an amount of intracellular target antigen bound to the antigen-binding polypeptide are detected using a Western blotting method. Next, by comparing the amount of intracellular target antigen per detected antigen-binding polypeptide between the case where the polypeptide of the present embodiment has been attached and the case where the polypeptide of the present embodiment has not been attached, and by confirming whether or not the amount of binding to the intracellular target antigen is increased in the case where the polypeptide of the present embodiment has been attached compared to the case where the polypeptide of the present embodiment has not been attached, it is possible to evaluate whether or not the binding of the antigen-binding polypeptide to an antigen within cells is improved.

For example, the amount of binding of an antigen-binding polypeptide to which the polypeptide of the present embodiment has been attached at its N-terminus to an intracellular target antigen can be, but is not limited to, for example, 1.2-fold, 1.5-fold, 1.8-fold, 2-fold, 3-fold, 4-fold, 5-fold, 6-fold, 7-fold, 8-fold, 9-fold, 10-fold, or more, relative to the amount of binding of an antigen-binding polypeptide to which the polypeptide of the present embodiment has not been attached to an intracellular target antigen.

Further, as shown in Experimental Example 12 described below, in cells expressing an antigen polypeptide, cell proliferation is suppressed compared to cells not expressing an antigen polypeptide; however, a fusion polypeptide in which the polypeptide of the present embodiment has been attached to the N-terminus of an antigen-binding polypeptide can reduce suppression of cell proliferation compared to the case where the antigen polypeptide alone is expressed within cells. Furthermore, compared to a case where a fusion polypeptide in which a polypeptide described in Patent Document 1 or the like, which is known to enhance intracellular antibody stability, has been attached to the N-terminus of an antigen polypeptide is expressed within cells, a fusion polypeptide in which the polypeptide of the present embodiment has been attached to the N-terminus of an antigen-binding polypeptide can reduce suppression of cell proliferation. That is, the polypeptide of the present embodiment can also be characterized as being capable of imparting an effect of reducing suppression of cell proliferation upon intracellular expression of an antigen-binding polypeptide.

The effect of reducing suppression of cell proliferation can be evaluated, for example, using the following method. That is, first, an expression vector containing a nucleic acid encoding an antigen-binding polypeptide to which the polypeptide of the present embodiment has been attached at its N-terminus, and an expression vector containing only a nucleic acid encoding an antigen-binding polypeptide to which the polypeptide of the present embodiment has not been attached, are prepared, and these expression vectors are each introduced into cells and expressed within the cells. Further, as a control, untreated cells into which no vector has been introduced are prepared. Next, the cells are cultured while being imaged over time using Incucyte S3 (Sartorius). After 40 hours from start of the culture, cell confluence (%) in a culture vessel is calculated using an Incucyte analysis module. Next, by comparing the cell confluence (%) after 40 hours from the start of culture between the case where the polypeptide of the present embodiment has been attached and the case where the polypeptide of the present embodiment has not been attached, and by confirming whether or not the cell confluence (%) is higher in the case where the polypeptide of the present embodiment has been attached than in the case where the polypeptide of the present embodiment has not been attached, it is possible to evaluate whether or not there is an effect of reducing suppression of cell proliferation. Further, with respect to a rate of decrease in cell confluence (%) relative to the cell confluence (%) of untreated cells into which no vector has been introduced, by confirming whether or not the rate of decrease is smaller in the case where the polypeptide of the present embodiment has been attached than in the case where the polypeptide of the present embodiment has not been attached, it is possible to evaluate whether or not there is an effect of reducing suppression of cell proliferation.

For example, the cell confluence (%) of cells expressing, within cells, a fusion polypeptide in which the polypeptide of the present embodiment has been attached to the N-terminus of an antigen-binding polypeptide can be, but is not limited to, for example, 1.1 -fold, 1.2-fold, 1.5-fold, 1.8-fold, 2-fold, or more, relative to the cell confluence (%) of cells expressing the antigen polypeptide alone within cells.

Further, for example, in cells expressing, within cells, a fusion polypeptide in which the polypeptide of the present embodiment has been attached to the N-terminus of an antigen-binding polypeptide, a rate of decrease {(1 - Y/X)×100 (%)} in cell confluence Y (%) relative to cell confluence X (%) of untreated cells can be, but is not limited to, for example, 40%, 30%, 20%, 10%, or less.

In the following, the polypeptide of the present embodiment is described in detail.

The polypeptide of the present embodiment has 3 or more amino acids, preferably 4 or more, more preferably 5 or more, still more preferably 6 or more, further preferably 7 or more, even more preferably 8 or more, and particularly preferably 9 or more amino acids. On the other hand, the polypeptide of the present embodiment has 20 or less, 19 or less, 18 or less, 17 or less, 16 or less, 15 or less, 14 or less, 13 or less, 12 or less, 11 or less, or 10 or less amino acids.

When the number of amino acids in the polypeptide of the present embodiment is not less than the above lower limit and not more than the above upper limit, binding to an intracellular target antigen can be improved without significantly reducing an expression level of a fusion polypeptide of the polypeptide of the present embodiment and an antigen-binding polypeptide within cells. On the other hand, when the number of amino acids in the polypeptide of the present embodiment is less than the lower limit, binding of a fusion polypeptide of the polypeptide of the present embodiment and an antigen-binding polypeptide to an intracellular target is reduced. Further, when the number of amino acids in the polypeptide of the present embodiment exceeds the upper limit, a risk of a decrease in an expression level of a fusion polypeptide of the polypeptide of the present embodiment and an antigen-binding polypeptide within cells is increased. Therefore, when the number of amino acids in the polypeptide of the present embodiment is within the above numerical range, a fusion polypeptide of the polypeptide of the present embodiment and an antigen-binding polypeptide does not significantly reduce its expression level within cells, and a proportion of antigen-binding polypeptides maintaining correct folding within cells is increased, and as a result, binding of an antigen-binding polypeptide to an antigen within cells can be improved.

That is, the polypeptide of the present embodiment has, for example, 3 or more and 20 or less, 4 or more and 20 or less, 5 or more and 20 or less, 6 or more and 20 or less, 7 or more and 20 or less, 8 or more and 20 or less, or 9 or more and 20 or less amino acids.

Further, the polypeptide of the present embodiment has, for example, 3 or more and 19 or less, 4 or more and 19 or less, 5 or more and 19 or less, 6 or more and 19 or less, 7 or more and 19 or less, 8 or more and 19 or less, or 9 or more and 19 or less amino acids.

Further, the polypeptide of the present embodiment has, for example, 3 or more and 18 or less, 4 or more and 18 or less, 5 or more and 18 or less, 6 or more and 18 or less, 7 or more and 18 or less, 8 or more and 18 or less, or 9 or more and 18 or less amino acids.

Further, the polypeptide of the present embodiment has, for example, 3 or more and 17 or less, 4 or more and 17 or less, 5 or more and 17 or less, 6 or more and 17 or less, 7 or more and 17 or less, 8 or more and 17 or less, or 9 or more and 17 or less amino acids.

Further, the polypeptide of the present embodiment has, for example, 3 or more and 16 or less, 4 or more and 16 or less, 5 or more and 16 or less, 6 or more and 16 or less, 7 or more and 16 or less, 8 or more and 16 or less, or 9 or more and 16 or less amino acids.

Further, the polypeptide of the present embodiment has, for example, 3 or more and 15 or less, 4 or more and 15 or less, 5 or more and 15 or less, 6 or more and 15 or less, 7 or more and 15 or less, 8 or more and 15 or less, or 9 or more and 15 or less amino acids.

Further, the polypeptide of the present embodiment has, for example, 3 or more and 14 or less, 4 or more and 14 or less, 5 or more and 14 or less, 6 or more and 14 or less, 7 or more and 14 or less, 8 or more and 14 or less, or 9 or more and 14 or less amino acids.

Further, the polypeptide of the present embodiment has, for example, 3 or more and 13 or less, 4 or more and 13 or less, 5 or more and 13 or less, 6 or more and 13 or less, 7 or more and 13 or less, 8 or more and 13 or less, or 9 or more and 13 or less amino acids.

Further, the polypeptide of the present embodiment has, for example, 3 or more and 12 or less, 4 or more and 12 or less, 5 or more and 12 or less, 6 or more and 12 or less, 7 or more and 12 or less, 8 or more and 12 or less, or 9 or more and 12 or less amino acids.

Further, the polypeptide of the present embodiment has, for example, 3 or more and 11 or less, 4 or more and 11 or less, 5 or more and 11 or less, 6 or more and 11 or less, 7 or more and 11 or less, 8 or more and 11 or less, or 9 or more and 11 or less amino acids.

Further, the polypeptide of the present embodiment has, for example, 3 or more and 10 or less, 4 or more and 10 or less, 5 or more and 10 or less, 6 or more and 10 or less, 7 or more and 10 or less, 8 or more and 10 or less, or 9 or more and 10 or less amino acids.

Among these, the polypeptide of the present embodiment preferably has 9 or more and 20 or less, more preferably 9 or more and 18 or less, still more preferably 9 or more and 15 or less, particularly preferably 9 or more and 12 or less, and most preferably 9 amino acids.

Examples of amino acids in the polypeptide of the present embodiment include those exemplified in the above definition of terms. Among these, the polypeptide of the present embodiment preferably has L-amino acids.

The polypeptide of the present embodiment has acidic amino acids as major constituents. Specifically, more than 57.0% and less than 100.0%, preferably 60.0% or more and 90.0% or less, more preferably 65.0% or more and 80.0% or less, still more preferably 66.0% or more and 78.0% or less, and particularly preferably 66.7% or more and 77.8% or less of the amino acids in the polypeptide of the present embodiment are acidic amino acids.

When the proportion of acidic amino acids relative to the amino acids in the polypeptide of the present embodiment exceeds the above lower limit or is equal to or greater than the above lower limit, binding of a fusion polypeptide of the polypeptide of the present embodiment and an antigen-binding polypeptide to an intracellular target antigen can be improved. On the other hand, when the proportion of acidic amino acids relative to the amino acids in the polypeptide of the present embodiment is less than the above upper limit or is equal to or less than the above upper limit, binding of a of a fusion polypeptide of the polypeptide of the present embodiment and an antigen-binding polypeptide to an intracellular target is improved without significantly reducing an expression level of the fusion polypeptide within cells. Therefore, when the proportion of acidic amino acids relative to the amino acids in the polypeptide of the present embodiment is within the above numerical range, a fusion polypeptide of the polypeptide of the present embodiment and an antigen-binding polypeptide does not significantly reduce its expression level within cells, and a proportion of antigen-binding polypeptides maintaining correct folding within cells is increased, and as a result, binding of an antigen-binding polypeptide to an antigen within cells can be improved.

As used herein, the proportion of a specific amino acid relative to the amino acids in a polypeptide is expressed as a percentage (%) calculated by dividing the number of the specific amino acid in the polypeptide by the total number of amino acids in the polypeptide and multiplying the result by 100.

Examples of acidic amino acids in the polypeptide of the present embodiment include those exemplified in the above definition of terms. Among these, the polypeptide of the present embodiment preferably has a glutamic acid at the N-terminus.

The polypeptide of the present embodiment preferably has acidic amino acids and neutral amino acids.

More than 0.0% and 40.0% or less, preferably 10.0% or more and 40.0% or less, more preferably 20.0% or more and 35.0% or less, still more preferably 22.0% or more and 34.0% or less, and particularly preferably 22.2% or more and 33.3% or less of the amino acids in the polypeptide of the present embodiment are neutral amino acids.

When the proportion of neutral amino acids relative to the amino acids in the polypeptide of the present embodiment is equal to or greater than the above lower limit, binding to an intracellular target antigen can be improved without significantly reducing an expression level of a fusion polypeptide of the polypeptide of the present embodiment and an antigen-binding polypeptide within cells. On the other hand, when the proportion of neutral amino acids relative to the amino acids in the polypeptide of the present embodiment is equal to or less than the above upper limit, binding of a fusion polypeptide of the polypeptide of the present embodiment and an antigen-binding polypeptide to an intracellular target is improved. Therefore, when the proportion of neutral amino acids relative to the amino acids in the polypeptide of the present embodiment is within the above numerical range, a proportion of antigen-binding polypeptides maintaining correct folding within cells is increased, and as a result, binding of an antigen-binding polypeptide to an antigen within cells can be further improved.

Examples of neutral amino acids in the polypeptide of the present embodiment include those exemplified in the above definition of terms. Among these, the neutral amino acids preferably have a side chain that is a hydrophobic aliphatic group or a hydrophilic group, more preferably are proline, glycine, or serine, and particularly preferably are proline.

Preferably 0.0% or more and 40.0% or less, more preferably 10.0% or more and 40.0% or less, still more preferably 20.0% or more and 35.0% or less, particularly preferably 22.0% or more and 34.0% or less, and most preferably 22.2% or more and 33.3% or less of the amino acids in the polypeptide of the present embodiment are proline.

When the proportion of proline relative to the amino acids in the polypeptide of the present embodiment is equal to or greater than the above lower limit, binding to an intracellular target antigen can be improved without significantly reducing an expression level of a fusion polypeptide of the polypeptide of the present embodiment and an antigen-binding polypeptide within cells. On the other hand, when the proportion of proline relative to the amino acids in the polypeptide of the present embodiment is equal to or less than the above upper limit, binding of a fusion polypeptide of the polypeptide of the present embodiment and an antigen-binding polypeptide to an intracellular target is improved. Therefore, when the proportion of proline relative to the amino acids in the polypeptide of the present embodiment is within the above numerical range, a proportion of antigen-binding polypeptides maintaining correct folding within cells is increased, and as a result, binding of an antigen-binding polypeptide to an antigen within cells can be further improved.

Alternatively, a polypeptide in another preferred embodiment further has basic amino acids in addition to acidic amino acids. The polypeptide of the present embodiment may have acidic amino acids and basic amino acids, or may have acidic amino acids, neutral amino acids, and basic amino acids.

Examples of acidic amino acids in the polypeptide of the present embodiment include those exemplified in the above definition of terms. Among these, the polypeptide of the present embodiment preferably has a glutamic acid at the N-terminus.

Examples of neutral amino acids in the polypeptide of the present embodiment include those exemplified in the above definition of terms. Among these, the neutral amino acids preferably have a side chain that is a hydrophobic aliphatic group or a hydrophilic group, more preferably are proline, glycine, or serine, and particularly preferably are proline.

Examples of basic amino acids in the polypeptide of the present embodiment include those exemplified in the above definition of terms. Among these, the basic amino acids are preferably lysine.

That is, the polypeptide of the present embodiment preferably further has basic amino acids, and the basic amino acids are lysine. Alternatively, the polypeptide of the present embodiment preferably has acidic amino acids, neutral amino acids, and basic amino acids, wherein the acidic amino acids are glutamic acid or aspartic acid, the neutral amino acids are proline, and the basic amino acids are lysine. Alternatively, the polypeptide of the present embodiment preferably has acidic amino acids, neutral amino acids, and basic amino acids, wherein the acidic amino acids are glutamic acid or aspartic acid, the neutral amino acids are proline, the basic amino acids are lysine, and its N-terminus is glutamic acid. Alternatively, the polypeptide of the present embodiment preferably has acidic amino acids and basic amino acids, wherein the acidic amino acids are glutamic acid or aspartic acid, and the basic amino acids are lysine. Alternatively, the polypeptide of the present embodiment preferably has acidic amino acids and basic amino acids, wherein the acidic amino acids are glutamic acid or aspartic acid, the basic amino acids are lysine, and its N-terminus is glutamic acid.

A total proportion of neutral amino acids and basic amino acids relative to the amino acids in the polypeptide of the present embodiment is preferably 10.0% or more and 40.0% or less, more preferably 20.0% or more and 35.0% or less, still more preferably 22.0% or more and 34.0% or less, and particularly preferably 22.2% or more and 33.3% or less.

When the total proportion of neutral amino acids and basic amino acids relative to the amino acids in the polypeptide of the present embodiment is equal to or greater than the above lower limit, binding to an intracellular target antigen can be improved without significantly reducing an expression level of the polypeptide of the present embodiment and an antigen-binding polypeptide within cells. On the other hand, when the total proportion of neutral amino acids and basic amino acids relative to the amino acids in the polypeptide of the present embodiment is equal to or less than the above upper limit, binding of a fusion polypeptide of the polypeptide of the present embodiment and an antigen-binding polypeptide to an intracellular target is improved. Therefore, when the total proportion of neutral amino acids and basic amino acids relative to the amino acids in the polypeptide of the present embodiment is within the above numerical range, a proportion of antigen-binding polypeptides maintaining correct folding within cells is increased, and as a result, binding of an antigen-binding polypeptide to an antigen within cells can be further improved.

Preferably 0.0% or more and 11.1% or less of the amino acids in the polypeptide of the present embodiment are neutral amino acids, and particularly preferably proline. When the proportion of neutral amino acids, preferably proline, relative to the amino acids in the polypeptide of the present embodiment is within the above numerical range, binding of an antigen-binding polypeptide to an antigen within cells can be further improved.

Preferably 11.1% or more and 22.2% or less of the amino acids in the polypeptide of the present embodiment are basic amino acids, particularly preferably lysine. When the proportion of basic amino acids, preferably lysine, relative to the amino acids in the polypeptide of the present embodiment is within the above numerical range, binding of an antigen-binding polypeptide to an antigen within cells can be further improved.

Examples of a polypeptide in a more preferred embodiment include a polypeptide having an amino acid sequence represented by the following general formula (I) (hereinafter also referred to as "polypeptide (I)"):

[Chemical Formula 1] (EX¹D)ₙ₁EX²X³(EX⁴D)ₙ₂ (I )

In general formula (I),
X¹ and X⁴ are each independently a neutral amino acid or a basic amino acid;
X² is D or a neutral amino acid;
X³ is D or E; and
n1 and n2 are each independently an integer of 0 or more and 3 or less, and 0 ≦ n1 + n2 ≦ 5.

Examples of neutral amino acids at X¹ and X⁴ include those exemplified in the above definition of terms.

Examples of basic amino acids at X¹ and X⁴ include those exemplified in the above definition of terms.

That is, X¹ and X⁴ in general formula (I) are each independently a neutral amino acid selected from a group consisting of alanine (A), asparagine (N), cysteine (C), phenylalanine (F), glycine (G), isoleucine (I), leucine (L), methionine (M), proline (P), glutamine (Q), serine (S), threonine (T), valine (V), tryptophan (W), and tyrosine (Y), or a basic amino acid selected from a group consisting of histidine (H), lysine (K), and arginine (R). Among these, X¹ and X⁴ in general formula (I) are each independently, preferably, a neutral amino acid having a side chain that is a hydrophobic aliphatic group or a hydrophilic group, or K, and more preferably G, P, S, or K.

In general formula (I), when n1 is 2 or more, multiple X¹ may be identical to different from each other.

In general formula (1), when n2 is 2 or more, multiple X⁴ may be identical to or different from each other.

Examples of neutral amino acids at X² include those exemplified in the above definitions of terms.

That is, X² in general formula (I) is D or a neutral amino acid selected from a group consisting of alanine (A), asparagine (N), cysteine (C), phenylalanine (F), glycine (G), isoleucine (I), leucine (L), methionine (M), proline (P), glutamine (Q), serine (S), threonine (T), valine (V), tryptophan (W), and tyrosine (Y). Among these, X² in general formula (I) preferably is D or a neutral amino acid having a side chain that is a hydrophobic aliphatic group or a hydrophilic group, more preferably D, G, P, or S, and still more preferably D.

In general formula (I), X³ is preferably E.

n1 and n2 are each independently preferably an integer of 0 or more and 3 or less, with 0 ≦ n1 + n2 ≦ 4; more preferably an integer of 0 or more and 3 or less, with 1 ≦ n1 + n2 ≦ 4; and still more preferably an integer of 0 or more and 3 or less, with 2 ≦ n1 + n2 ≦ 4.

Examples of preferred polypeptides (I) include those shown in the following table. These are merely examples of preferred polypeptides (I) and are not limiting.

**[Table 1]**

| SEQ ID NO | Amino Acid Sequence | Peptide Length |
|---|---|---|
| - | EX²X³ | 3 |
| - | EX¹DEX²X³ | 6 |
| - | EX²X³EX⁴D | 6 |
| 64 | EX^{1D}EX²X³EX⁴D | 9 |
| 65 | EX¹DEX¹DEX²X³ | 9 |
| 66 | EX²X³EX⁴DEX⁴D | 9 |
| 67 | EX¹DEX¹DEX²X³EX⁴D | 12 |
| 68 | EX¹DEX²X³EX DEX⁴D | 12 |
| 69 | EX¹DEX¹DEX¹DEX²X³ | 12 |
| 70 | EX²X³EX⁴DEX⁴DEX⁴D | 12 |
| 71 | EX¹DEX¹DEX¹DEX²X³EX⁴D | 15 |
| 72 | EX¹DEX¹DEX²X³EX⁴DEX⁴D | 15 |
| 73 | EX¹DEX²X³EX⁴DEX⁴DEX⁴D | 15 |
| 74 | EX¹DEX¹DEX¹DEX¹DEX²X³ | 15 |
| 75 | EX²X³EX⁴DEX⁴DEX⁴DEX⁴D | 15 |
| 76 | EX¹DEX¹DEX¹DEX¹DEX²X³EX⁴D | 18 |
| 77 | EX¹DEX DEX¹DEX²X³EX⁴DEX⁴D | 18 |
| 78 | EX¹DEX¹DEX²X³EX⁴DEX⁴DEX⁴D | 18 |
| 79 | EX¹DEX²X³EX⁴DEX⁴DEX⁴DEX⁴D | 18 |
| 80 | EX¹DEX¹DEX¹DEX¹DEX¹DEX²X³ | 18 |
| 81 | EX²X³EX⁴DEX⁴DEX⁴DEX⁴DEX⁴D | 18 |

Among these, preferred polypeptides (I) include a polypeptide having an amino acid sequence EX²X³, a polypeptide having an amino acid sequence EX¹DEX²X³, a polypeptide having an amino acid sequence EX²X³EX⁴D, or a polypeptide having an amino acid sequence represented by any one of SEQ ID NOs: 64 to 66 and 72, and more preferred polypeptides (I) include a polypeptide having an amino acid sequence represented by any one of SEQ ID NOs: 64 to 66 and 72.

Alternatively, examples of a polypeptide in another more preferred embodiment include a polypeptide having an amino acid sequence represented by the following general formula (II) (hereinafter also referred to as "polypeptide (II)"):

[Chemical Formula 2] (EX¹DEX²X³EX⁴D)ₙ₃ EX⁵(EX¹DEX²X³EX⁴D)ₙ₄ (11)

In general formula (II),
X¹, X², X³, and X⁴ are the same as those in the above general formula (I);
X⁵ is a neutral amino acid or a basic amino acid; and
n3 and n4 are each independently an integer of 0 or more and 1 or less, and satisfy a relational expression: 1 ≦ n3 + n4 ≦ 2.

Examples of neutral amino acids at X⁵ include those exemplified in the above definitions of terms.

Examples of basic amino acids at X⁵ include those exemplified in the above definition of terms.

That is, X⁵ in general formula (II) is a neutral amino acid selected from a group consisting of alanine (A), asparagine (N), cysteine (C), phenylalanine (F), glycine (G), isoleucine (I), leucine (L), methionine (M), proline (P), glutamine (Q), serine (S), threonine (T), valine (V), tryptophan (W), and tyrosine (Y), or a basic amino acid selected from a group consisting of histidine (H), lysine (K), and arginine (R). Among these, X⁵ in general formula (II) is preferably a neutral amino acid having a side chain that is a hydrophobic aliphatic group or a hydrophilic group, or K, and more preferably G, P, S, or K.

n3 and n4 are each independently preferably 1, and n3 + n4 = 2.

**[Table 2]**

| SEQ ID NO | Amino Acid Sequence | Peptide Length |
|---|---|---|
| 18 | EX¹DEX²X³EX⁴DEX⁵ | 11 |
| 38 | EX³EX¹DEX²X³EX⁴D | 11 |
| 82 | EX¹DEX²X³EX¹DEX⁵EX¹DEX²X³EX¹D | 20 |

Among these, a preferred polypeptide (II) is a polypeptide having an amino acid sequence represented by SEQ ID NO: 82.

Specific examples of polypeptides in a still more preferred embodiment include, but are not limited to, those shown in the following table.

**[Table 3]**

| SEQ ID NO | Peptide No. | Amino Acid Sequence | Peptide Length | Acidic Amino Acid Content (%) | Neutral Amino Acid Content (%) |
|---|---|---|---|---|---|
| 23 | peptide 1 | EPDEDEEPDEPEPDEDEEPD | 20 | 75. 0 | 25.0 |
| 24 | peptide 2 | EPDEPDEDEEPDEPD | 15 | 73.4 | 26. 6 |
| 25 | peptide 3 | EPDEDEEPD | 9 | 77.8 | 22. 2 |
| 31 | peptide 9 | EGDEDEEGD | 9 | 77.8 | 22.2 |
| 32 | peptide 10 | EFDEDEEFD | 9 | 77. 8 | 22.2 |
| 33 | peptide 11 | ESDEDEESD | 9 | 77.8 | 22.2 |
| 34 | peptide 12 | EPDEPDEPD | 9 | 77. 8 | 33.3 |
| - | peptide 16 | EPD | 3 | 66. 7 | 33.3 |
| 39 | peptide 17 | EPDEPD | 6 | 66. 7 | 33. 3 |
| 40 | peptide 18 | EKDEDEEKD | 9 | 77.8 | 0.0 |
| 41 | peptide 19 | EKDEDEEPD | 9 | 77.8 | 11. 1 |
| 42 | peptide 20 | EPDEDEEKD | 9 | 77. 8 | 11. 1 |

Among these, the polypeptide of the present embodiment is preferably a polypeptide having an amino acid sequence represented by any one of SEQ ID NOs: 23 to 25, 31, 33, 34, and 40 to 42. Further, the polypeptide of the present embodiment is particularly preferably a polypeptide having an amino acid sequence represented by SEQ ID NO: 25.

### [Nucleic Acid Encoding Polypeptide]

In one embodiment, the present disclosure provides a nucleic acid encoding the above polypeptide.

The nucleic acid of the present embodiment may be a DNA molecule, an RNA molecule, or a hybrid molecule of DNA and RNA. Further, the nucleic acid of the present embodiment may be single-stranded or double-stranded.

The nucleic acid of the present embodiment can be prepared using known genetic engineering techniques or chemical synthesis methods, or the like. A specific nucleotide sequence of the nucleic acid of the present embodiment can be readily designed by a person skilled in the art, for example with reference to a codon table, based on the amino acid sequence of the above polypeptide, preferably an amino acid sequence EX²X³, an amino acid sequence EX¹DEX²X³, or an amino acid sequence EX²X³EX⁴D, or an amino acid sequence represented by any one of SEQ ID NOs: 64 to 66, 72, and 82, and more preferably an amino acid sequence represented by any of SEQ ID NOs: 23 to 25, 31, 33, 34, and 40 to 42.

Specific examples of nucleic acids encoding the preferred polypeptides include those shown in the following table.

**[Table 4]**

| SEQ ID NO | Peptide No. | Nucleotide Sequence |
|---|---|---|
| 3 | peptide 1 | GAGCCAGACGAGGATGAGGAACCTGACGAACCCGAACCAGACGAAGATGAAGAACCGGAT |
| 4 | peptide 2 | GAGCCCGATGAACCCGACGAGGACGAAGAGCCAGATGAACCGGAC |
| 5 | peptide 3 | GAACCTGATGAAGATGAAGAACCTGAT |
| 11 | peptide 9 | GAGGGAGATGAGGATGAGGAGGGCGAC |
| 12 | peptide 10 | GAATTCGACGAGGATGAAGAGTTTGAC |
| 13 | peptide 11 | GAGAGCGATGAGGACGAGGAGTCCGAC |
| 14 | peptide 12 | GAACCCGATGACCCTGACGAACCTGAT |
| | peptide 16 | GAACCTGAT |
| 19 | peptide 17 | GAACCTGATGAGCCTGAC |
| 20 | peptide 18 | GAAAAGGATGAAGATGAAGAAAAGGAT |
| 21 | peptide 19 | GAAAAGGATGAAGATGAAGAACCTGAT |
| 22 | peptide 20 | GAACCTGATGAAGATGAAGAAAAGGAT |

Among these, the nucleic acid encoding the polypeptide of the present embodiment is preferably a nucleic acid having a nucleotide sequence represented by any one of SEQ ID NOs: 3 to 5, 11, 13, 14, 19, and 20 to 22. Further, the nucleic acid encoding the polypeptide of the present embodiment is particularly preferably a nucleic acid having a nucleotide sequence represented by SEQ ID NO: 5.

### <Fusion Polypeptide>

A fusion polypeptide of the present embodiment includes the above polypeptide and an antigen-binding polypeptide. Alternatively, the fusion polypeptide of the present embodiment can also be described as including an antigen-binding polypeptide and the above polypeptide attached to the N-terminus of the antigen-binding polypeptide.

In the fusion polypeptide of the present embodiment, by having the above polypeptide attached to the N-terminus of the antigen-binding polypeptide, a proportion of antigen-binding polypeptides maintaining correct folding within cells is increased, and as a result, binding of the antigen-binding polypeptide to an antigen within cells can be further improved.

### [Antigen-Binding Polypeptide]

Examples of antigen-binding polypeptides include those exemplified in the above definitions of terms.

It is known that, since an intracellular environment is in a more reductive state compared to an extracellular environment, conventional antibodies requiring disulfide bond formation for structural formation are difficult to express. Therefore, the antigen-binding polypeptide is preferably one that is stably expressed and functions even in such a reductive intracellular environment. Therefore, in particular, the antigen-binding polypeptide is preferably a monoclonal antibody against a specific intracellular antigen, and more preferably one that includes one or more complementarity-determining regions (CDRs) of an antibody against a specific intracellular antigen. Specific examples of such antibodies include those including at least one of heavy chain CDR1, heavy chain CDR2, heavy chain CDR3, light chain CDR1, light chain CDR2, and light chain CDR3 of any monoclonal antibody. Preferably, such antibodies include at least two, at least three, at least four, at least five, or all of these CDRs.

Among these, preferred antigen-binding polypeptides include scFv, VH, VL, VHH, vNAR, and Fab for a specific antigen.

### [Other Polypeptides]

The fusion polypeptide of the present embodiment may further include other polypeptides at the C-terminus of the fusion polypeptide. In the fusion polypeptide of the present embodiment, a linker sequence having about 1 or more and 10 or less amino acids may be included between the above antigen-binding polypeptide and the other polypeptides. Examples of linker sequences include, but are not limited to, a linker sequence having about 1 or more and 10 or less, preferably about 2 or more and 5 or less glycines.

When the fusion polypeptide of the present embodiment is used for pharmaceutical applications, it is preferable to use, among the other polypeptides, a pharmaceutically acceptable polypeptide, or alternatively, not to include such other polypeptides.

Examples of other polypeptides include purification tag peptides, detection peptides, degradation-promoting peptides, Neh2 domain peptides, stress-responsive degradation peptides, drug-binding peptides, and the like.

Examples of purification tag peptides include known tag peptides such as HA tags and His tags.

Detection peptides are not particularly limited, and examples thereof include, but are not limited to, fluorescent proteins, enzymes that produce light emission or color change upon reaction, and the like.

Examples of fluorescent proteins include, but are not limited to, BFP, EBFP, CFP, ECFP, Cypet, AmCyan1, GFP, EGFP, YFP, Venus, mKO, mOrange, RFP, DsRed, tdTomato, mCherry, mStrawberry, Azalea, mPlum, mAG, Kaede, Dronpa, Keima, KikG, KikGR, UnaG, and the like.

Examples of enzymes that produce light emission or color change upon reaction include, but are not limited to, peroxidase, alkaline phosphatase, β-D-galactosidase, glucose oxidase, glucose-6-phosphate dehydrogenase, alcohol dehydrogenase, malate dehydrogenase, penicillinase, catalase, apoglucose oxidase, urease, luciferase, acetylcholinesterase, and the like.

Examples of degradation-promoting peptides include, but are not limited to, HSC70-binding peptides, XIAP RING domain peptides, and the like.

Examples of Neh2 domain peptides include, but are not limited to, peptides derived from Nrf2, and the like.

Examples of stress-responsive degradation peptides include, but are not limited to, oxygen-dependent degradation domains represented by peptides derived from HIF1α, and the like.

### [Nucleic Acid Encoding the Fusion Polypeptide]

In one embodiment, the present disclosure provides a nucleic acid encoding the above fusion polypeptide.

The nucleic acid encoding the above fusion polypeptide may be a DNA molecule, an RNA molecule, or a hybrid molecule of DNA and RNA. Further, the nucleic acid encoding the above fusion polypeptide may be single-stranded or double-stranded.

The nucleic acid encoding the above fusion polypeptide can be prepared using known genetic engineering techniques or chemical synthesis methods, or the like. A specific nucleotide sequence of the nucleic acid of the present embodiment can be readily designed by a person skilled in the art, for example with reference to a codon table, based on the amino acid sequence of the above fusion polypeptide.

The nucleic acid encoding the above fusion polypeptide may include a non-coding nucleic acid having one or more bases between a nucleic acid encoding the above polypeptide and a nucleic acid encoding the above antigen-binding polypeptide, and/or between a nucleic acid encoding the above antigen-binding polypeptide and a nucleic acid encoding the above other polypeptides. The number of bases included in the non-coding nucleic acid may be, for example, 1 or more and 100 or less, 1 or more and 50 or less, 1 or more and 30 or less, or 1 or more and 10 or less.

### <Expression Vector>

An expression vector of the present embodiment includes a nucleic acid encoding the above polypeptide.

Alternatively, an expression vector in another embodiment includes a nucleic acid encoding the above fusion polypeptide.

In the present embodiment, examples of expression vectors into which a nucleic acid encoding the above polypeptide or a nucleic acid encoding the above fusion polypeptide is inserted include those exemplified in the above definitions of terms.

The expression vector can be constructed, for example, using known genetic engineering techniques.

### <Pharmaceutical Composition>

In one embodiment, the present disclosure provides a pharmaceutical composition including a nucleic acid encoding the above fusion polypeptide or an expression vector including the nucleic acid, and a pharmaceutically acceptable carrier.

Examples of pharmaceutically acceptable carriers include, when the pharmaceutical composition of the present embodiment is formulated as an aqueous solution, pure water such as sterile water, physiological saline, phosphate-buffered saline, and the like. When the pharmaceutical composition of the present embodiment is formulated as another suitable solution, examples include organic esters that can be introduced into a living body, such as glycol, glycerol, and olive oil.

The pharmaceutical composition of the present embodiment can further include other components such as lubricants, preservatives, stabilizers, wetting agents, emulsifiers, salts for osmotic pressure adjustment, buffering agents, excipients, antioxidants, viscosity modifiers, colorants, flavoring agents, and sweeteners.

The pharmaceutical composition of the present embodiment may be contained in a container, pack, dispenser, or the like together with instructions for use.

### [Pharmaceutical Applications]

The pharmaceutical composition of the present embodiment can be used, for example, to treat or prevent a disease associated with an intracellular antigen.

In one embodiment, the pharmaceutical composition of the present disclosure introduces a nucleic acid encoding the above fusion polypeptide or an expression vector including the nucleic acid into cells of a subject, expresses the above fusion polypeptide in the cells of the subject, and regulates, that is, suppresses or activates, and preferably suppresses, a function of an antigen associated with a disease by the antigen-binding polypeptide, thereby treating or preventing the disease.

One aspect of "treatment" encompasses, with respect to at least one symptom associated with a target disease, alleviation or mitigation, delaying progression, and curing. One aspect of "prevention" encompasses, with respect to at least one symptom associated with a target disease, preventing onset.

Examples of subjects of treatment and prevention include humans and non-human animals. More specifically, examples of non-human animals include vertebrates such as fish, birds, and mammals. Examples of mammals include laboratory animals such as mice, rats, rabbits, guinea pigs, and non-human primates; companion animals (pets) such as dogs and cats; and livestock such as pigs, cattle, goats, sheep, and horses.

Examples of diseases to be treated or prevented include cancer, tumors, infectious diseases (such as viral infections and bacterial infections), autoimmune diseases or allergic diseases, inflammatory diseases, neurodegenerative diseases, and the like.

A route of administration and a method of administration of the pharmaceutical composition of the present embodiment are not particularly limited and can be appropriately selected depending on a target disease. The pharmaceutical composition may be directly administered to an affected area or may be indirectly administered. Further, cells expressing the above fusion polypeptide may be administered. In one example, examples of routes of administration include oral, intrathecal, intracisternal, intracerebroventricular, intravenous, intramuscular, subcutaneous, intratumoral, rectal, intraarterial, intraportal, intraventricular, transmucosal, transdermal, intranasal, intraperitoneal, intrapulmonary, and intrauterine routes, as well as local administration and administration using a gene gun.

Dose and frequency of administration can be appropriately selected depending on symptom severity, age, sex, body weight, dosage form, specific type of disease, and the like.

### <Research Reagent>

In one embodiment, the present disclosure provides a research reagent including a nucleic acid encoding the above polypeptide or an expression vector including the nucleic acid. The research reagent may further include other components in addition to the nucleic acid encoding the above polypeptide or the expression vector including the nucleic acid. Such other components may be selected with reference to those described for the above pharmaceutical composition.

The research reagent of the present embodiment may be contained in a container, pack, dispenser, or the like together with instructions for use.

### [Use as Research Tool]

The research reagent of the present embodiment can be used in analyzing a function of an antigen in cells, for example, a polypeptide. In one example, functional analysis of an antigen can be performed by expressing, within cells, a fusion polypeptide of an antigen-binding polypeptide against the antigen to be functionally analyzed and the above polypeptide, and regulating, preferably inhibiting, the function of the antigen.

Target cells are not particularly limited. Examples of target cells include human cells and non-human animal cells. More specifically, examples of non-human animal cells include vertebrate cells such as fish cells, bird cells, and mammalian cells. Examples of mammalian cells include cells derived from laboratory animals such as mice, rats, rabbits, guinea pigs, and non-human primates; companion animals (pets) such as dogs and cats; and livestock such as pigs, cattle, goats, sheep, and horses.

Further, the cells may be cultured cells or in vivo cells, that is, non-isolated cells present in a living body. Preferred examples of cells include human cultured cells, human in vivo cells, cultured cells of a disease model non-human animal, and in vivo cells of a disease model non-human animal.

When introducing a viral vector into target cells, for example, a method of suspending the viral vector in a cell culture medium can be used. Introduction of a non-viral vector into target cells can be performed, for example, using various methods, such as electroporation, microinjection, lipofection, a calcium phosphate method, and a DEAE-dextran method.

Examples of indicators of results of inhibiting the function of the antigen include changes in phenotypes that appear in cells, tissues, or individuals, and functional analysis of the antigen can be performed based on these indicators. Examples of indicators of cellular changes include changes in cellular phenotypes, such as quantitative and/or qualitative changes in produced substances, changes in proliferative activity, changes in cell number, changes in morphology, changes in characteristics, and induction of apoptosis. As produced substances, secreted proteins, surface antigens, intracellular proteins, mRNA, and the like can be used. As morphological changes, formation of protrusions and/or changes in the number of protrusions, changes in flatness, changes in elongation/aspect ratio, changes in cell size, changes in internal structure, heterogeneity/homogeneity of cell populations, changes in cell density, and the like can be used. These morphological changes can be confirmed by observation under a microscope. As changes in characteristics, anchorage dependence, cytokine-dependent responsiveness, hormone dependence, drug resistance, cell motility, cell migration activity, pulsatility, changes in intracellular substances, and the like can be used. Examples of cell motility include cell invasion activity and cell migration activity. Further, as changes in intracellular substances, enzyme activity, mRNA levels, levels of intracellular signaling substances such as Ca²⁺ or cAMP, intracellular protein levels, and the like can be used. As indicators of tissue systems, functional changes corresponding to tissue used can be used as detection indicators. As indicators of biological systems, changes in tissue weight, changes in blood systems such as changes in blood cell count, changes in protein levels, enzyme activity, and electrolyte levels, as well as changes in the circulatory system such as changes in blood pressure and heart rate, and the like can be used.

Methods for measuring these detection indicators are not particularly limited, and absorbance, luminescence, colorimetry, fluorescence, radioactivity, fluorescence polarization, surface plasmon resonance signals, time-resolved fluorescence, mass, absorption spectra, light scattering, fluorescence resonance energy transfer, and the like can be used. These measurement methods are well known to those skilled in the art and can be appropriately selected depending on a purpose. For example, absorption spectra can be measured using commonly used photometers, plate readers, or the like, luminescence using luminometers or the like, and fluorescence using fluorometers or the like. Mass can be measured using a mass spectrometer. Radioactivity can be measured using measuring an instrument such as a gamma counter depending on the type of radiation, fluorescence polarization using BEACON (Takara Bio), surface plasmon resonance signal using BIACORE (GE Healthcare), and time-resolved fluorescence, fluorescence resonance energy transfer, and the like using ARVO (PerkinElmer) or the like. Furthermore, a flow cytometer and the like can also be used for measurement. These measurement methods may be used to measure two or more detection indicators with a single measurement method, and when convenient, it is also possible to measure a larger number of detection indicators by performing two or more measurements simultaneously and/or sequentially. For example, fluorescence and fluorescence resonance energy transfer can be measured simultaneously using a fluorometer.

The research reagent of the present embodiment can also be used for observing dynamics of an antigen in cells, for example, a polypeptide. In one example, dynamics of an antigen can be observed by expressing, within a cell, a fusion polypeptide of an antigen-binding polypeptide against the antigen to be observed and the above polypeptide, and detecting a reaction between the antigen and the fusion polypeptide. Observing dynamics of an antigen encompasses observing expression of the antigen, localization of the antigen, and the like, at a given time or over time.

Detection of the interaction between the antigen and the fusion polypeptide can be performed using, for example, fluorescence, luminescence, color development, or the like produced by a detection peptide included in the fusion polypeptide.

### <Method for Expressing Antigen-Binding Polypeptide in Cells>

In one embodiment, the present disclosure provides a method for expressing an antigen-binding polypeptide in cells. The expression method includes a step of expressing, in cells, a nucleic acid encoding a fusion polypeptide of the above polypeptide and an antigen-binding polypeptide.

Expression may be constitutive or transient, and may also be inducible. For constitutive expression, a nucleic acid in which a promoter for constitutive expression is linked upstream of a gene of the fusion polypeptide may be used. For transient expression, for example, RNA rather than DNA may be introduced into cells for expression. For inducible expression, a nucleic acid in which an inducible promoter is linked upstream of a gene encoding the fusion polypeptide may be used. When expressing the antigen-binding polypeptide, an inducer, such as IPTG, may be introduced into cells.

Examples of target cells include those listed above for the research reagent.

In the expression method of the present embodiment, the antigen-binding polypeptide is expressed in a state fused with the above polypeptide, that is, as a fusion polypeptide. By including the above polypeptide, the fusion polypeptide increases a proportion of antigen-binding polypeptides maintaining correct folding within cells compared to a case where the antigen-binding polypeptide is expressed alone. Therefore, within cells, binding of the antigen-binding polypeptide to an antigen is improved compared to the case where the antigen-binding polypeptide is expressed alone.

The expression method of the present embodiment further includes a step of introducing into cells a nucleic acid encoding the above-described fusion polypeptide. The nucleic acid encoding the above-described fusion polypeptide may be introduced in a form of an expression vector. Specific examples of introduction methods include those described for the above research reagent. Accordingly, further, in one embodiment, the present disclosure provides a method for producing cells that express an antigen-binding polypeptide in cells. The production method includes a step of introducing into cells a nucleic acid encoding the above-described fusion polypeptide or an expression vector including the nucleic acid. Further, in one embodiment, the present disclosure also provides cells expressing the above-described fusion polypeptide.

### <Kit>

In one embodiment, the present disclosure provides a kit for preparing a nucleic acid encoding a fusion polypeptide including the above-described polypeptide and an antigen-binding polypeptide, the kit including a nucleic acid encoding the polypeptide.

The kit of the present embodiment can be a versatile kit that enables easy preparation of a nucleic acid encoding a fusion polypeptide for a desired antigen-binding polypeptide.

The nucleic acid encoding the above-described polypeptide may be incorporated into an expression vector such as that described above. Further, the nucleic acid may include, for example, factors necessary for protein expression, such as a promoter, a ribosome-binding site, and a terminator, as well as a selection marker, a restriction enzyme recognition site, and the like.

The kit of the present embodiment may further include at least one of a buffer, a restriction enzyme, other necessary reagents, instruments, instructions for use, and the like.

### <Supplementary Notes>

The present disclosure can also include the following aspects.
(1) A polypeptide for improving binding of an antigen-binding polypeptide to an antigen within cells, the polypeptide comprising 3 or more and 20 or less amino acids, wherein more than 57.0% and less than 100.0% of the amino acids are acidic amino acids.
(2) The polypeptide according to (1), comprising 9 or more and 20 or less amino acids.
(3) The polypeptide according to (1) or (2), wherein 60.0% or more and 90.0% or less of the amino acids are acidic amino acids.
(4) The polypeptide according to any one of (1) to (3), wherein 65.0% or more and 80.0% or less of the amino acids are acidic amino acids.
   (4-1) The polypeptide according to any one of (1) to (4), wherein 66.0% or more and 78.0% or less of the amino acids are acidic amino acids.
   (4-2) The polypeptide according to any one of (1) to (4-1), wherein 66.7% or more and 77.8% or less of the amino acids are acidic amino acids.
(5) The polypeptide according to any one of (1) to (4-2), having glutamic acid at its N-terminus.
(6) The polypeptide according to any one of (1) to (5), comprising acidic amino acids and neutral amino acids.
(7) The polypeptide according to (6), wherein 10.0% or more and 40.0% or less of the amino acids are neutral amino acids.
(8) The polypeptide according to (6) or (7), wherein 20.0% or more and 35.0% or less of the amino acids are neutral amino acids.
   (8-1) The polypeptide according to any one of (6) to (8), wherein 22.0% or more and 34.0% or less of the amino acids are neutral amino acids.
   (8-2) The polypeptide according to any one of (6) to (8-1), wherein 22.2% or more and 33.3% or less of the amino acids are neutral amino acids.
(9) The polypeptide according to any one of (6) to (8-2), wherein side chains of the neutral amino acids are each a hydrophobic aliphatic group or a hydrophilic group.
(10) The polypeptide according to any one of (6) to (9), wherein the neutral amino acids are proline, glycine, or serine.
   (10-1) The polypeptide according to any one of (6) to (10), wherein the neutral amino acids are proline.
   (10-2) The polypeptide according to (10-1), wherein more than 0.0% and 40.0% or less of the amino acids are proline.
   (10-3) The polypeptide according to (10-1) or (10-2), wherein 10.0% or more and 40.0% or less of the amino acids are proline.
   (10-4) The polypeptide according to any one of (10-1) to (10-3), wherein 20.0% or more and 35.0% or less of the amino acids are proline.
   (10-5) The polypeptide according to any one of (10-1) to (10-4), wherein 22.0% or more and 34.0% or less of the amino acids are proline.
   (10-6) The polypeptide according to any one of (10-1) to (10-5), wherein 22.2% or more and 33.3% or less of the amino acids are proline.
(11) The polypeptide according to any one of (1) to (5), further comprising basic amino acids, wherein the basic amino acids are lysine.
   (11-1) The polypeptide according to (11), further comprising neutral amino acids, wherein a total proportion of neutral amino acids and basic amino acids relative to the amino acids is 10.0% or more and 40.0% or less.
   (11-2) The polypeptide according to (11) or (11-1), wherein the total proportion of neutral amino acids and basic amino acids relative to the amino acids is 20.0% or more and 35.0% or less.
   (11-3) The polypeptide according to any one of (11) to (11-2), wherein the total proportion of neutral amino acids and basic amino acids relative to the amino acids is 22.0% or more and 34.0% or less.
   (11-4) The polypeptide according to any one of (11) to (11-3), wherein the total proportion of neutral amino acids and basic amino acids relative to the amino acids is 22.2% or more and 33.3% or less.
   (11-5) The polypeptide according to any one of (11) to (11-4), wherein more than 0.0% and 11.1% or less of the amino acids are neutral amino acids.
   (11-6) The polypeptide according to any one of (11) to (11-5), wherein 0.0% or more and 11.1 % or less of the amino acids are proline.
   (11-7) The polypeptide according to any one of (11) to (11-6), wherein 11.1% or more and 22.2% or less of the amino acids are basic amino acids.
   (11-8) The polypeptide according to any one of (11) to (11-7), wherein 11.1% or more and 22.2% or less of the amino acids are lysine.
(12) The polypeptide according to any one of (1) to (11-8), comprising an amino acid sequence represented by the following general formula (I):

   [Chemical Formula 3] (EX¹D)ₙ₁(EX⁴D)ₙ₂ (I)

   In general formula (I),
   X¹ and X⁴ are each independently a neutral amino acid or a basic amino acid;
   X² is D or a neutral amino acid;
   X³ is D or E; and
   n1 and n2 are each independently an integer of 0 or more and 3 or less, and 0 ≦ n1 + n2 ≦ 5.

   (12-1) A polypeptide for improving binding of an antigen-binding polypeptide to an antigen within cells, the polypeptide comprising an amino acid sequence represented by the following general formula (I).

      [Chemical Formula 4] (EX¹D)n₁EX²X³(EX⁴D)ₙ₂ (I)

      In general formula (I),
   X¹ and X⁴ are each independently a neutral amino acid or a basic amino acid;
   X² is D or a neutral amino acid;
   X³ is D or E; and
   n1 and n2 are each independently an integer of 0 or more and 3 or less, and 0 ≦ n1 + n2 ≦ 5.
   (12-2) The polypeptide according to (12) or (12-1), wherein X¹ and X⁴ are each independently a neutral amino acid having a side chain that is a hydrophobic aliphatic group or a hydrophilic group, or lysine.
   (12-3) The polypeptide according to any one of (12) to (12-2), wherein X¹ and X⁴ are each independently glycine, proline, serine, or lysine.
   (12-4) The polypeptide according to any one of (12) to (12-3), wherein X² is aspartic acid, or a neutral amino acid having a side chain that is a hydrophobic aliphatic group or a hydrophilic group.
   (12-5) The polypeptide according to any one of (12) to (12-4), wherein X² is aspartic acid, glycine, proline, or serine.
   (12-6) The polypeptide according to any one of (12) to (12-5), wherein X² is aspartic acid.
   (12-7) The polypeptide according to any one of (12) to (12-6), wherein X³ is glutamic acid.
   (12-8) The polypeptide according to any one of (12) to (12-7), wherein n1 and n2 satisfy 0 ≤ n1 + n2 ≤ 4.
   (12-9) The polypeptide according to any one of (12) to (12-8), wherein n1 and n2 satisfy 1 ≤ n1 + n2 ≤ 4.
   (12-10) The polypeptide according to any one of (12) to (12-9), wherein n1 and n2 satisfy 2 ≤ n1 + n2 ≤ 4.
   (12-11) The polypeptide according to any one of (12) to (12-10), comprising an amino acid sequence selected from a group consisting of EX²X³, EX¹DEX²X³,EX²X³EX⁴D, and an amino acid sequence represented by any one of SEQ ID NOs: 64 to 66 and 72.
   (12-12) The polypeptide according to any one of (12) to (12-11), comprising an amino acid sequence represented by any one of SEQ ID NOs: 64 to 66 and 72.
(13) The polypeptide according to any one of (1) to (11-8), comprising an amino acid sequence represented by the following general formula (II):

   [Chemical Formula 5] (EX¹DEX²X³EX⁴D)ₙ₃ EX⁵(EX¹DEX²X³EX⁴D)ₙ₄ (II)

   In general formula (II),
   X¹ and X⁴ are each independently a neutral amino acid or a basic amino acid;
   X² is D or a neutral amino acid;
   X³ is D or E;
   X⁵ is a neutral amino acid or a basic amino acid; and
   n3 and n4 are each independently an integer of 0 or more and 1 or less, and satisfy a relational expression: 1 ≦ n3 + n4 ≦ 2.
   (13-1) A polypeptide for improving binding of an antigen-binding polypeptide to an antigen within cells, the polypeptide comprising an amino acid sequence represented by the following general formula (II).

      [Chemical Formula 6] (EX¹DEX²X³EX⁴D)ₙ₃EX⁵(EX¹DEX²X³EX⁴D)ₙ₄ (II)

      In general formula (II),
   X¹ and X⁴ are each independently a neutral amino acid or a basic amino acid;
   X² is D or a neutral amino acid;
   X³ is D or E;
   X⁵ is a neutral amino acid or a basic amino acid; and
   n3 and n4 are each independently an integer of 0 or more and 1 or less, and satisfy a relational expression: 1 ≦ n3 + n4 ≦ 2.
   (13-2) The polypeptide according to (13) or (13-1), wherein X¹ and X⁴ are each independently a neutral amino acid having a side chain that is a hydrophobic aliphatic group or a hydrophilic group, or lysine.
   (13-3) The polypeptide according to any one of (13) to (13-2), wherein X¹ and X⁴ are each independently glycine, proline, serine, or lysine.
   (13-4) The polypeptide according to any one of (13) to (13-3), wherein X² is aspartic acid, or a neutral amino acid having a side chain that is a hydrophobic aliphatic group or a hydrophilic group.
   (13-5) The polypeptide according to any one of (13) to (13-4), wherein X² is aspartic acid, glycine, proline, or serine.
   (13-6) The polypeptide according to any one of (13) to (13-5), wherein X² is aspartic acid.
   (13-7) The polypeptide according to any one of (13) to (13-6), wherein X³ is glutamic acid.
   (13-8) The polypeptide according to any one of (13) to (13-7), wherein X⁵ is a neutral amino acid having a side chain that is a hydrophobic aliphatic group or a hydrophilic group, or lysine.
   (13-9) The polypeptide according to any one of (13) to (13-8), wherein X⁵ is glycine, proline, serine, or lysine.
   (13-10) The polypeptide according to any one of (13) to (13-9), wherein n3 and n4 are 1.
   (13-11) The polypeptide according to any one of (13) to (13-10), comprising an amino acid sequence represented by SEQ ID NO: 82.
(14) The polypeptide according to any one of (1) to (13-11), comprising an amino acid sequence represented by any one of SEQ ID NOs: 23 to 25, 31, 33, 34, and 40 to 42.
   (14-1) A polypeptide for improving binding of an antigen-binding polypeptide to an antigen within cells, the polypeptide comprising an amino acid sequence represented by any one of SEQ ID NOs: 23 to 25, 31, 33, 34, and 40 to 42.
   (14-2) The polypeptide according to any one of (1) to (14-1), comprising an amino acid sequence represented by SEQ ID NO: 25.
   (14-3) A polypeptide for improving binding of an antigen-binding polypeptide to an antigen within cells, the polypeptide comprising an amino acid sequence represented by SEQ ID NO: 25.
(15) A fusion polypeptide comprising the polypeptide according to any one of (1) to (14-3) and an antigen-binding polypeptide.
(16) The fusion polypeptide according to (15), wherein the antigen-binding polypeptide has one or more complementarity-determining regions of an antibody against a specific antigen.
(17) The fusion polypeptide according to (15) or (16), wherein the antigen-binding polypeptide is Fab, VH, VL, VHH, vNAR, or scFv.
   (17-1) The fusion polypeptide according to any one of (15) to (17), wherein the antigen-binding polypeptide is VH, VHH, vNAR, or scFv.
(18) A nucleic acid encoding the polypeptide according to any one of (1) to (14-3).
   (18-1) The nucleic acid according to (18), comprising a nucleotide sequence represented by any one of SEQ ID NOs: 3 to 5, 11, 13, 14, 19, and 20 to 22.
   (18-2) A nucleic acid encoding a polypeptide for improving binding of an antigen-binding polypeptide to an antigen within cells, the nucleic acid comprising a nucleotide sequence represented by any one of SEQ ID NOs: 3 to 5, 11, 13, 14, 19, and 20 to 22.
   (18-3) The nucleic acid according to any one of (18) to (18-2), comprising a nucleotide sequence represented by SEQ ID NO: 5.
   (18-4) A nucleic acid encoding a polypeptide for improving binding of an antigen-binding polypeptide to an antigen within cells, the nucleic acid comprising a nucleotide sequence represented by SEQ ID NO: 5.
(19) An expression vector comprising the nucleic acid according to any one of (18) to (18-4).
   (19-1) The expression vector according to (19), comprising a nucleic acid having a nucleotide sequence represented by any one of SEQ ID NOs: 3 to 5, 11, 13, 14, 19, and 20 to 22.
   (19-2) An expression vector comprising a nucleic acid encoding a polypeptide for improving binding of an antigen-binding polypeptide to an antigen within cells, wherein the nucleic acid has a nucleotide sequence represented by any one of SEQ ID NOs: 3 to 5, 11, 13, 14, 19, and 20 to 22.
   (19-3) The expression vector according to any one of (19) to (19-2), comprising a nucleic acid having a nucleotide sequence represented by SEQ ID NO: 5.
   (19-4) An expression vector comprising a nucleic acid encoding a polypeptide for improving binding of an antigen-binding polypeptide to an antigen within cells, wherein the nucleic acid has a nucleotide sequence represented by SEQ ID NO: 5.
(20) A nucleic acid encoding the fusion polypeptide according to any one of (15) to (17).
(21) An expression vector comprising the nucleic acid according to (20).
(22) A method for improving binding of an antigen-binding polypeptide to an antigen within cells, comprising
   expressing, in cells, a nucleic acid encoding a fusion polypeptide, wherein
   the fusion polypeptide includes the antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (22-1) A method for improving binding of an antigen-binding polypeptide to an antigen within cells, comprising
   expressing, in cells, a nucleic acid encoding a fusion polypeptide, wherein
   the fusion polypeptide includes the antigen-binding polypeptide and the polypeptide according to any one of (1) to (14-3) linked to the N-terminus of the antigen-binding polypeptide.
(23) A method for improving folding efficiency of an antigen-binding polypeptide within cells, comprising
   expressing, in cells, a nucleic acid encoding a fusion polypeptide, wherein
   the fusion polypeptide includes the antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (23-1) A method for improving folding efficiency of an antigen-binding polypeptide within cells, comprising
   expressing, in cells, a nucleic acid encoding a fusion polypeptide, wherein
   the fusion polypeptide includes the antigen-binding polypeptide and the polypeptide according to any one of (1) to (14-3) linked to the N-terminus of the antigen-binding polypeptide.
(24) A method for increasing a proportion of antigen-binding polypeptides maintaining correct folding within cells, comprising
   expressing, in cells, a nucleic acid encoding a fusion polypeptide, wherein
   the fusion polypeptide includes the antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (24-1) A method for increasing a proportion of antigen-binding polypeptides maintaining correct folding within cells, comprising
   expressing, in cells, a nucleic acid encoding a fusion polypeptide, wherein
   the fusion polypeptide includes the antigen-binding polypeptide and the polypeptide according to any one of (1) to (14-3) linked to the N-terminus of the antigen-binding polypeptide.
(25) A method for treating or preventing a disease associated with an intracellular antigen, comprising
   expressing, in cells, a nucleic acid encoding a fusion polypeptide, wherein
   the fusion polypeptide includes the antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (25-1) A method for treating or preventing a disease associated with an intracellular antigen, comprising
   expressing, in cells, a nucleic acid encoding a fusion polypeptide, wherein
   the fusion polypeptide includes the antigen-binding polypeptide and the polypeptide according to any one of (1) to (14-3) linked to the N-terminus of the antigen-binding polypeptide.
   (25-2) The method according to any one of (22) to (25-1), further comprising introducing into cells a nucleic acid encoding the fusion polypeptide prior to the expressing.
(26) A fusion polypeptide for use in treating or preventing a disease associated with an intracellular antigen,
   the fusion polypeptide comprising an antigen-binding polypeptide and a polypeptide linked to the N-terminus of the antigen-binding polypeptide, wherein
   the polypeptide has 3 or more and 20 or less amino acids, and more than 57.0% and less than 100.0% of the amino acids in the polypeptide are acidic amino acids.
   (26-1) A fusion polypeptide for use in treating or preventing a disease associated with an intracellular antigen,
   comprising an antigen-binding polypeptide and the polypeptide according to any one of (1) to (14-3) linked to the N-terminus of the antigen-binding polypeptide.
(27) Use of a fusion polypeptide for manufacturing a pharmaceutical composition for treating or preventing a disease associated with an intracellular antigen, wherein
   the fusion polypeptide includes an antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (27-1) Use of a fusion polypeptide for manufacturing a pharmaceutical composition for treating or preventing a disease associated with an intracellular antigen, wherein
   the fusion polypeptide includes the antigen-binding polypeptide and the polypeptide according to any one of (1) to (14-3) linked to the N-terminus of the antigen-binding polypeptide.
(28) A nucleic acid for use in treating or preventing a disease associated with an intracellular antigen,
   the nucleic acid encoding a fusion polypeptide, wherein
   the fusion polypeptide includes an antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (28-1) A nucleic acid for use in treating or preventing a disease associated with an intracellular antigen,
   the nucleic acid encoding a fusion polypeptide, wherein
   the fusion polypeptide includes the antigen-binding polypeptide and the polypeptide according to any one of (1) to (14-3) linked to the N-terminus of the antigen-binding polypeptide.
(29) Use of a nucleic acid for manufacturing a pharmaceutical composition for treating or preventing a disease associated with an intracellular antigen, wherein
   the nucleic acid encodes a fusion polypeptide,
   the fusion polypeptide includes an antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (29-1) Use of a nucleic acid for manufacturing a pharmaceutical composition for treating or preventing a disease associated with an intracellular antigen, wherein
   the nucleic acid encodes a fusion polypeptide, and
   the fusion polypeptide includes the antigen-binding polypeptide and the polypeptide according to any one of (1) to (14-3) linked to the N-terminus of the antigen-binding polypeptide.
(30) The use according to any one of (28) to (29-1), wherein the nucleic acid is incorporated into an expression vector.
(31) A pharmaceutical composition for use in treating or preventing a disease associated with an intracellular antigen, comprising
   a nucleic acid encoding a fusion polypeptide or an expression vector including the nucleic acid; and a pharmaceutically acceptable carrier, wherein
   the fusion polypeptide includes an antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (31-1) A pharmaceutical composition for use in treating or preventing a disease associated with an intracellular antigen, comprising
   a nucleic acid encoding the fusion polypeptide according to any one of (15) to (17) or an expression vector including the nucleic acid; and a pharmaceutically acceptable carrier.
   (31-2) A pharmaceutical composition for use in treating or preventing a disease associated with an intracellular antigen, comprising
   a nucleic acid encoding a fusion polypeptide or an expression vector including the nucleic acid; and a pharmaceutically acceptable carrier, wherein
   the fusion polypeptide includes the antigen-binding polypeptide and the polypeptide according to any one of (1) to (14-3) linked to the N-terminus of the antigen-binding polypeptide.
(32) A research reagent for use in analyzing a function of an antigen within cells, comprising a nucleic acid encoding the polypeptide according to any one of (1) to (14-3) or an expression vector including the nucleic acid.
(33) A method for producing a cell expressing an antigen-binding polypeptide within cells, comprising
   expressing, in cells, a nucleic acid encoding a fusion polypeptide, wherein
   the fusion polypeptide includes an antigen-binding polypeptide and a polypeptide linked to the N-terminus of the antigen-binding polypeptide, and
   the polypeptide has 3 or more and 20 or less amino acids, and more than 57.0% and less than 100.0% of the amino acids in the polypeptide are acidic amino acids.
   (33-1) The method according to (33), further comprising introducing into cells a nucleic acid encoding the fusion polypeptide prior to the expressing.
(34) A kit for producing a nucleic acid encoding a fusion polypeptide, comprising a nucleic acid encoding the polypeptide according to any one of (1) to (14-3) or an expression vector including the nucleic acid.

### Examples

The present disclosure is described below by way of examples; however, the present disclosure is not limited to the following examples.

### [Antigen-Binding Polypeptide]

As an antigen-binding polypeptide, an intrabody (hereinafter referred to as "Y13-259-HA") based on an anti-KRAS antibody in the form of an scF, with an HA tag attached to the C-terminus side thereof (nucleotide sequence: SEQ ID NO: 1; amino acid sequence: SEQ ID NO: 2) (reference: Werge T. M., et al., Intracellular immunization: Cloning and intracellular expression of a monoclonal antibody to the p21ras protein, FEBS Letters 274, 193-198, 1990), was used.

Further, as an antigen-binding polypeptide that recognizes an antigen different from KRAS, an intrabody (nucleotide sequence: SEQ ID NO: 83; amino acid sequence: SEQ ID NO: 84) (hereinafter referred to as "scFv13-HA") having an anti-β-galactosidase antibody in the form of an scFv, with an HA tag attached to the C-terminus side thereof (reference: P. Martineau et al., Expression of an Antibody Fragment at High Levels in the Bacterial Cytoplasm, J. Mol. Biol. 280, 117-127, 1998), was used.

As an antigen-binding polypeptide in a format different from scFv, an intrabody (nucleotide sequence: SEQ ID NO: 85; amino acid sequence: SEQ ID NO: 86) (hereinafter referred to as "iDab#6-HA") having an anti-HRAS (G12V mutant) antibody in the form of a VH, with an HA tag attached to the C-terminus side thereof (reference: T. Tanaka et al., Tumour prevention by a single antibody domain targeting the interaction of signal transduction proteins with RAS, EMBO J. 26, 3250-3259, 2007), was used.

Further, as antigen-binding polypeptides that recognize antigens other than KRAS, HRAS, and β-galactosidase and have formats different from scFv and VH, an intrabody (nucleotide sequence: SEQ ID NO: 87; amino acid sequence: SEQ ID NO: 88) (hereinafter referred to as "BsG73-HA") having an anti-EGFP shark antibody (vNAR) (International Publication No. WO 2023/077287) with an HA tag attached to the C-terminus side thereof, and an intrabody (nucleotide sequence: SEQ ID NO: 89; amino acid sequence: SEQ ID NO: 90) (hereinafter referred to as "enhancer-HA") having an anti-EGFP camelid antibody (VHH (also referred to as a nanobody); reference PDB ID: 3K1K) with an HA tag attached to the C-terminus side thereof, were used.

### [Polypeptide for Improving Binding of an Antigen-Binding Polypeptide to an Antigen within Cells>]

Nucleotide sequences (SEQ ID NOs: 3 to 22) and amino acid sequences (SEQ ID NOs: 23 to 42) of peptides 1 to 20 used in the present Example are respectively shown in the following tables. Vectors for expressing antibodies in which these peptides were attached to the N-terminus of an antibody having an amino acid sequence of SEQ ID NO: 1 were constructed and used for various studies.

**[Table 5]**

| Nucleotide Sequences of Peptides 1-20 | | |
|---|---|---|
| SEQ ID NO | Peptide No. | Nucleotide Sequence |
| 3 | peptide 1 | GAGCCAGACGAGGATGAGGAACCTGACGAACCCGAACCAGACGAAGATGAAGAACCGGAT |
| 4 | peptide 2 | GAGCCCGATGAACCCGACGAGGACGAAGAGCCAGATGAACCGGAC |
| 5 | peptide 3 | GAACCTGATGAAGATGAAGAACCTGAT |
| 6 | peptide 4 | GACTACAAAGACCATGATGGCGACTATAAAGACCACGACATCGACTACAAAGACGACGATGACAA G |
| 7 | Peptide 5 | GATGAACAGGAGAACGATTATGATGAGCCAGAAGTGAACGACGAGAACCAGGATTATGATGAG |
| 8 | peptide 6 | GATGACGACGAGGACGAAGACGACGAAGATGAGGATGAAGATGAAGACGAGGATGAGGACGAGGA C |
| 9 | peptide 7 | AAACCCAGGAAACGGAAGAAGCCAAGA |
| 10 | peptide 8 | CAGCCAAACCAGAACCAACAGCCCAAT |
| 11 | peptide 9 | GAGGGAGATGAGGATGAGGAGGGCGAC |
| 12 | peptide 10 | GAATTCGACGAGGATGAAGAGTTTGAC |
| 13 | peptide 11 | GAGAGCGATGAGGACGAGGAGTCCGAC |
| 14 | peptide 12 | GAACCCGATGAGCCTGACGAACCTGAT |
| 15 | peptide 13 | GAGCCTCCTGAGCCTGACGAGCCGGAT |
| 16 | peptide 14 | GAGCCACCAGAGCCTCCCGAGCCAGAC |
| 17 | peptide 15 | GAACCTCCCGAACCACCCGAACCTCCC |
| - | peptide 16 | GAACCTGAT |
| 19 | peptide 17 | GAACCTGATGAGCCTGAC |
| 20 | peptide 18 | GAAAAGGATGAAGATGAAGAAAAGGAT |
| 21 | peptide 19 | GAAAAGGATGAAGATGAAGAACCTGAT |
| 22 | peptide 20 | GAACCTGATGAAGATGAAGAAAAGGAT |

**[Table 6]**

| Amino Acid Sequences of Peptides 1 to 20 | | | | | |
|---|---|---|---|---|---|
| SEQ ID NO | Pepti de No. | Amino Acid Sequence | Peptide Length | Prolin (P) Content (%) | Neutral Amino Acid Content (%) |
| 23 | peptide 1 | EPDEDEEPDEPEPDEDEEPD | 20 | 25.0 | 25.0 |
| 24 | peptide 2 | EPDEPDEDEEPDEPD | 15 | 26.6 | 26.6 |
| 25 | peptide 3 | EPDEDEEPD | 9 | 22.2 | 22.2 |
| 26 | peptide 4 | DYKDIIDGDYKDIIDIDYKDDDDK | 22 | 0. 0 | 22.7 |
| 27 | Peptide 5 | DEQENDYDEPEV\DENQDYDE | 21 | 4.8 | 42.9 |
| 28 | peptide 6 | DDDEDEDDEDEDEDEDEDEDED | 22 | 0. 0 | 0. 0 |
| 29 | peptide 7 | KPRKRKKPR | 9 | 22.2 | 22.2 |
| 30 | peptide 8 | QPNQNQQPN | 9 | 22.2 | 22.2 |
| 31 | peptide 9 | EGDEDEEGD | 9 | 0. 0 | 22.2 |
| 32 | peptide 10 | EFDEDEEFD | 9 | 0. 0 | 22.2 |
| 33 | peptide 11 | ESDEDEESD | 9 | 0. 0 | 22.2 |
| 34 | peptide 12 | EPDEPDEPD | 9 | 33.3 | 33.3 |
| 35 | peptide 13 | EPPEPDEPD | 9 | 44.4 | 44. 4 |
| 36 | peptide 14 | EPPEPPEPD | 9 | 55.5 | 55. 5 |
| 37 | peptide 15 | EPPEPPEPP | 9 | 66.6 | 66. 6 |
| - | peptide 16 | EPD | 3 | 33.3 | 33.3 |
| 39 | peptide 17 | EPDEPD | 6 | 33. 3 | 33. 3 |
| 40 | peptide 18 | EKDEDEEKD | 9 | 0. 0 | 0. 0 |
| 41 | peptide 19 | EKDEDEEPD | 9 | 11. 1 | 11. 1 |
| 42 | peptide 20 | EPDEDEEKD | 9 | 11. 1 | 11. 1 |

### [Antigen Protein]

As an antigen protein recognized by Y13-259-HA, a KRAS protein having a His tag attached to its C-terminus side (nucleotide sequence: SEQ ID NO: 43; amino acid sequence: SEQ ID NO: 44) (hereinafter referred to as "KRAS-His") was used.

Further, as an antigen protein recognized by scFv13-HA, a β-galactosidase protein having a FLAG tag attached to its C-terminus (nucleotide sequence: SEQ ID NO: 91; amino acid sequence: SEQ ID NO: 92) (hereinafter referred to as "β-Gal-FLAG") was used.

Further, as an antigen protein recognized by iDab#6-HA, an HRAS (G12V) protein having a FLAG tag attached to its C-terminus (nucleotide sequence: SEQ ID NO: 93; amino acid sequence: SEQ ID NO: 94) (hereinafter referred to as "HRAS (G12V)-FLAG") was used.

Further, as an antigen protein recognized by BsG73-HA and enhancer-HA, EGFP (nucleotide sequence: SEQ ID NO: 95; amino acid sequence: SEQ ID NO: 96) was used.

### [Construction of Expression Vector]

First, Y13-259-HA (nucleotide sequence: SEQ ID NO: 1), peptide 3 (nucleotide sequence: SEQ ID NO: 5)-Y13-259-HA (nucleotide sequence: SEQ ID NO: 1), and KRAS-His (nucleotide sequence: SEQ ID NO: 43) were each prepared by artificial gene synthesis and inserted into a multiple cloning site of pcDNA3.4 (Invitrogen) using genetic engineering techniques, thereby constructing expression vectors. Next, using a pcDNA3.4 expression vector into which Y13-259-HA had been inserted as a template, gene fragments of Y13-259-HA to which a peptide other than peptide3 had been attached were amplified by PCR using primers shown in the the following table, and each expression vector was constructed using an In-Fusion method. A sequence cloned into each expression vector is a sequence in which a nucleotide sequence represented by any one of SEQ ID NOs: 3 to 22 was linked to a nucleotide sequence represented by SEQ ID NO: 1.

**[Table 7]**

| Primer Sequences of Peptides | | |
|---|---|---|
| SEQ ID NO | peptide No. | Nucleotide Sequence of Primer |
| 45 | peptide 1 | GCTTGCCGCCACCATGGAGCCAGACGAGGATGAGGAACCTGACGAACCCGAACCAGACGAAGA TGAAGAACCGGATCAGGTGCAGTTGCAGCAAAGTGG |
| 46 | peptide 2 | GCTTGCCGCCACCATGGAGCCCGATGAACCCGACGAGGACGAAGAGCCAGATGAACCGGACCA GGTGCAGTTGCAGCAAAGTGGAG |
| 47 | peptide 1 | ATGACAAGCAGGTGCAGTTGCAGCAAAGTGGAG |
| 48 | peptide 5 | ATGATGAGCAGGTGCAGTTGCAGCAAAGTGGAG |
| 49 | peptide 6 | GCTTGCCGCCACCATGGATGACGACGAGGACGAAGACGACGAAGATGAGGATGAAGATGAAGA CGAGGATGAGGACGAGGACCAGGTGCAGTTGCAGCAAAGTGG |
| 50 | peptide 7 | GCTTGCCGCCACCATGAAACCCAGGAAACGGAAGAAGCCAAGACAGGTGCAGTTGCAGCAAAG TGGAG |
| 51 | peptide 8 | GCTTGCCGCCACCATGCAGCCAAACCAGAACCAACAGCCCAATCAGGTGCAGTTGCAGCAAAG TGGAG |
| 52 | peptide 9 | GCTTGCCGCCACCATGGAGGGAGATGAGGATGAGGAGGGCGACCAGGTGCAGTTGCAGCAAAG TGGAG |
| 53 | peptide 10 | GCTTGCCGCCACCATGGAATTCGACGAGGATGAAGAGTTTGACCAGGTGCAGTTGCAGCAAAG TGGAG |
| 54 | peptide 11 | GCTTGCCGCCACCATGGAGAGCGATGAGGACGAGGAGTCCGACCAGGTGCAGTTGCAGCAAAG TGGAG |
| 55 | peptide 12 | GCTTGCCGCCACCATGGAACCCGATGAGCCTGACGAACCTGATCAGGTGCAGTTGCAGCAAAG TGGAG |
| 56 | peptide 13 | GCTTGCCGCCACCATGGAGCCTCCTGAGCCTGACGAGCCGGATCAGGTGCAGTTGCAGCAAAG TGGAG |
| 57 | peptide 14 | GCTTGCCGCCACCATGGAGCCACCAGAGCCTCCCGAGCCAGACCAGGTGCAGTTGCAGCAAAG TGGAG |
| 58 | peptide 15 | GCTTGCCGCCACCATGGAACCTCCCGAACCACCCGAACCTCCCCAGGTGCAGTTGCAGCAAAG TGGAG |
| 59 | peptide 16 | GCTTGCCGCCACCATGGAACCTGATCAGGTGCAGTTGCAGCAAAGTGGAG |
| 60 | peptide 17 | GCTTGCCGCCACCATGGAACCTGATGAGCCTGACCAGGTGCAGTTGCAGCAAAGTGGAG |
| 61 | peptide 18 | GCTTGCCGCCACCATGGAAAAGGATGAAGATGAAGAAAAGGATCAGGTGCAGTTGCAGCAAAG TGGAG |
| 62 | peptide 19 | GCTTGCCGCCACCATGGAAAAGGATGAAGATGAAGAACCTGATCAGGTGCAGTTGCAGCAAAG TGGAG |
| 63 | peptide 20 | GCTTGCCGCCACCATGGAACCTGATGAAGATGAAGAAAAGGATCAGGTGCAGTTGCAGCAAAG TGGAG |

Expression vectors other than those described above were constructed by GeneArt Service (Thermo Fisher Scientific) using a pcDNA3.4 vector.

### [Immunoprecipitation]

In Experimental Examples 1 to 6, 293A cells cultured in a 96-well plate were co-transfected with an expression vector for an antigen protein and an expression vector for an antigen-binding polypeptide. Transfection of plasmid DNA was performed using FuGENE HD Transfection Reagent (Promega) according to an attached protocol. Three days after transfection, the cells were collected and lysed with 40 µL of Radioimmunoprecipitation (RIPA) buffer (Nacalai Tesque). The lysed cell solution was centrifuged at 4 °C and 10,000 × g for 10 minutes to prepare approximately 40 µL of cell lysate. To 15 µL of each cell lysate, 75 µL of Phosphate-buffered saline (PBS) buffer was added, and the resulting mixture was combined with 5 µL of anti-HA-tag monoclonal antibody-magnetic agarose (Medical & Biological Laboratories) and the mixture was allowed to react at 4 °C for 1 hour to recover an antibody expressed in the cells. After the reaction, a supernatant was removed using a magnet, and the magnetic agarose was washed four times with PBS-T buffer (0.1 v/v% Tween 20). After washing, 12 µL of Western blotting buffer was added to the magnetic agarose, followed by heat treatment at 100 °C for 10 minutes, and a supernatant was collected using a magnet.

In Experimental Example 7 (Immunoprecipitation of endogenous KRAS) and Experimental Example 9 (Immunoprecipitation of HRAS (G12V)), cells were cultured using a 6-well plate. The cells were lysed with 200 µL of RIPA buffer, and a mixture prepared by adding 160 µL of PBS buffer to 60 µL of each prepared cell lysate was combined with 20 µL of anti-HA-tag magnetic agarose. After washing, in Experimental Example 8, 24 µL of Western blotting buffer was added to the magnetic agarose.

In Experimental Examples 9 to 11, the transfection reagent was changed to FuGENE 4K Transfection Reagent (Promega), and procedure was performed using a reverse transfection method. Further, the washing buffer was also changed to PBS-T buffer (0.01 v/v% Tween 20).

In Experimental Example 8 (Immunoprecipitation of β-Gal-FLAG) and Experimental Examples 10 and 11 (Immunoprecipitation of EGFP), immunoprecipitation was performed by adding 80 µL of PBS buffer to 20 µL of the prepared cell lysate.

In Experimental Example 7 (immunoprecipitation of endogenous KRAS), transfection was performed using a pcDNA3.4_Y13-259-HA expression vector alone, without peptide attachment or with pep3 attached. In Experimental Examples 8 to 11, co-transfection was performed using various pcDNA3.4_intrabody-HA expression vectors without peptide attachment or with pep3 attached, together with pcDNA3.4 vectors for corresponding target antigens.

Experimental Example 8 was conducted with N = 6. Experimental Examples 1 to 7 and 9 to 11 were conducted with N = 3.

### [Western blotting]

10 µL of each cell lysate prepared by immunoprecipitation was subjected to electrophoresis on a 4-20 w/w% Mini-PROTEAN TGX Stain-Free^{™} gel (Bio-Rad) (280 V, 15-20 minutes). Further, similarly, 10 µL of the supernatant collected after immunoprecipitation was subjected to electrophoresis on a 4-20 w/w% Mini-PROTEAN TGX Precast Protein Gel (Bio-Rad). After electrophoresis, the samples were transferred onto a low-fluorescence polyvinylidene difluoride (PVDF) membrane (Bio-Rad) using Trans-Blot Turbo (Bio-Rad), and the membrane was blocked using EveryBlot blocking buffer (Bio-Rad). After blocking, to detect Y13-259-HA and KRAS-His, a 1:2500 dilution of HA-Tag (C29F4) rabbit monoclonal antibody (Cell Signaling Technology) and a 1:5000 dilution of 6×His, His-Tag monoclonal antibody (Proteintech Group Inc.) were reacted as primary antibodies for 1 hour. After the reaction, the membrane was washed with Tris-buffered saline (TBS)-T buffer (0.1 v/v% Tween 20), and further reacted with a 1:5000 dilution of Goat Anti-Rabbit IgG (H+L), Highly Cross-Adsorbed (Biotium) and a 1:2500 dilution of StarBright Blue 700 Goat AntiMouse IgG (Bio-Rad). After the reaction, the membrane was washed with TBS-T buffer and imaged using ChemiDoc MP (Bio-Rad).

In Experimental Examples 6 to 11, the detection antibodies were changed to improve detection sensitivity. For detection of intrabodies, a 1:2,000 dilution of Anti-HA High Affinity Rat Antibody (Merck) was used as the primary antibody, and a 1:2,000 dilution of Goat Anti-Rat IgG (H+L) Cross-Adsorbed Secondary Antibody, DyLight^{™} 800 (Thermo Fisher Scientific) was used as the secondary antibody. For detection of various target antigens, the primary antibodies described below and a 1:2,500 dilution of StarBright Blue 700 Goat Anti-Rabbit IgG (Bio-Rad) as the secondary antibody were used.

For detection of the target antigen KRAS-His in Experimental Example 6, a 1:1,000 dilution of Anti-His-tag pAb (Medical & Biological Laboratories) was used as the primary antibody. For detection of endogenous intracellular KRAS, which was the target antigen in Experimental Example 7, a 1:10,000 dilution of KRAS Polyclonal antibody 12063-1-AP (Proteintech) was used as the primary antibody. For detection of β-Gal-FLAG, which was the target antigen in Experimental Example 8, and HRAS (G12V)-FLAG, which was the target antigen in Experimental Example 9, a 1:500 dilution of anti-FLAG^{®} rabbit antibody (Merck) was used. For detection of EGFP, which was the target antigen in Experimental Examples 10 and 11, a 1:2,000 dilution of eGFP Polyclonal Antibody (CAB4211, Thermo Fisher Scientific) was used.

Only in the Western blotting of Experimental Example 8, the amount of the supernatant sample after immunoprecipitation subjected to electrophoresis on the gel was set to 3 µL.

### [Cell Proliferation Assay]

In Experimental Example 12, 293A cells cultured in a 96-well half-area plate were co-transfected with a pcDNA3.4_KRAS-His expression vector and pcDNA3.4_Y13-259-HA expression vectors to which respective peptides had been attached. Transfection of plasmid DNA was performed using FuGENE 4K Transfection Reagent (Promega) by a reverse transfection method. After transfection, the cells in each well were incubated at 37 °C while being imaged over time using Incucyte S3 (Sartorius). Further, the confluence (%) of the 293A cells in each well was calculated using an Incucyte analysis module. The experiment was performed with N = 3.

### [Experimental Example 1]

### (Investigation of amino acid backbones in polypeptides for improving binding of antigen-binding polypeptides to antigens within cells)

In order to search for polypeptides capable of improving a binding function of an antigen-binding polypeptide to a target antigen within cells, first, as backbones of the polypeptides, peptides consisting of 9 amino acids mainly composed of acidic amino acids (peptide3), basic amino acids (peptide7), or neutral amino acids (peptide8) were each designed, and vectors expressing intrabodies in which each peptide was fused to the N-terminus side of Y13-259AHA were constructed. The three constructed intrabody expression vectors and the pcDNA3.4_KRAS-His expression vector were co-transfected into 293A cells, each intrabody expressed in the cells was recovered by immunoprecipitation, and the amounts of each intrabody and KRAS contained in the recovered samples were compared by Western blotting.

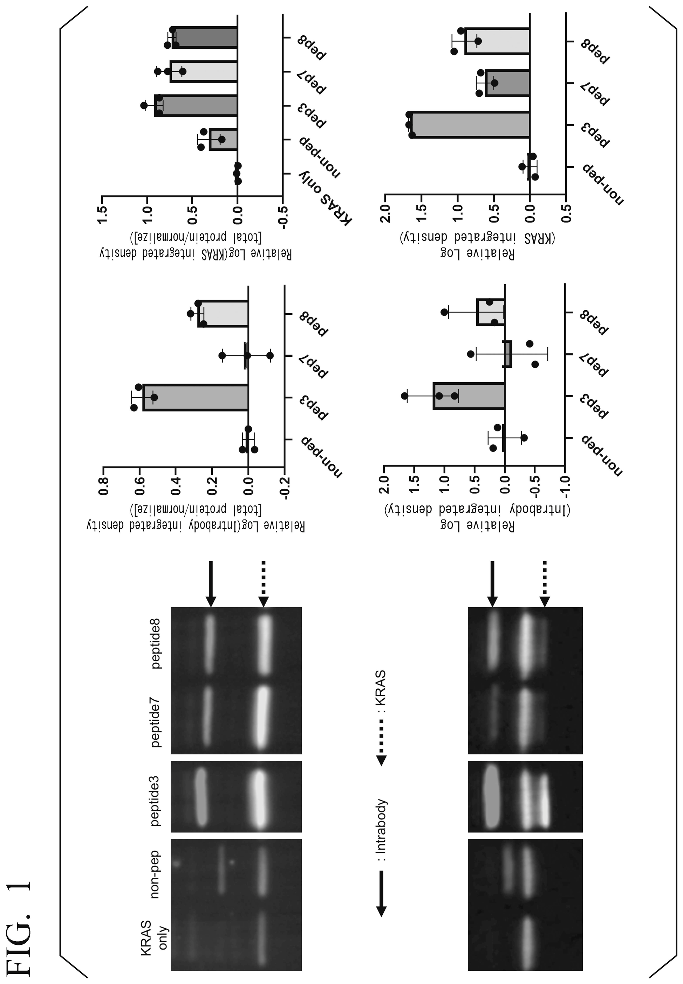

The upper panels of Fig. 1 show photographs and graphs presenting the analysis results of the cell lysates (samples before immunoprecipitation). The upper left panel of Fig. 1 shows photographs of the results of evaluation by Western blotting of the expression of each intrabody (solid arrow) and KRAS (dashed arrow) contained in the cell lysates before immunoprecipitation.

The upper center panel of Fig. 1 is a graph showing the results of comparing the ratios of expression levels of the respective intrabodies, in which the luminance value of each band was normalized by the total protein amount based on the results of Western blotting, the logarithmically transformed values were averaged, and the value for Y13-259-HA without peptide attachment was used as a reference. The vertical axis of the graph indicates the expression level (relative value) of the intrabody. As a result, compared to Y13-259-HA without peptide attachment (indicated as "non-pep" in the figure), Y13-259-HA with peptide3 composed of an acidic amino acid backbone attached (indicated as "Peptide3" or "pep3" in the figure) and Y13-259-HA with peptide8 composed of a neutral amino acid backbone attached (indicated as "Peptide8" or "pep8" in the figure) exhibited an increasing tendency in intrabody expression levels. On the other hand, Y13-259-HA with peptide7 composed of a basic amino acid backbone attached (indicated as "Peptide7" or "pep7" in the figure) showed no difference in expression level compared to Y13-259-HA without peptide attachment.

The upper right panel of Fig. 1 is a graph showing the results of comparing the KRAS expression levels, in which the luminance value of each band was normalized by the total protein amount based on the results of Western blotting, the logarithmically transformed values were averaged, and the value obtained when only KRAS was expressed (indicated as "KRAS only" in the figure) was used as a reference. The vertical axis of the graph indicates the expression level (relative value) of KRAS. As a result, co-expression with an intrabody showed a tendency to increase the amount of KRAS accumulated in the cytoplasm, and particularly in cases where a peptide had been attached, a tendency was observed for the amount of KRAS accumulated in the cytoplasm to be greater compared to cases where no peptide had been attached.

The lower panels of Fig. 1 show photographs and graphs presenting the analysis results of the samples after immunoprecipitation. The lower left panel of Fig. 1 shows photographs of the results of evaluation by Western blotting of the amounts of each intrabody (solid arrow) and KRAS (dashed arrow) in the samples after immunoprecipitation.

The lower center panel of Fig. 1 is a graph showing the results of comparing the amounts of the respective intrabodies, in which the luminance values of the respective intrabodies after immunoprecipitation were logarithmically transformed and then averaged based on the results of Western blotting, and the value for Y13-259-HA without peptide attachment was used as a reference. The vertical axis of the graph indicates the amount (relative value) of the intrabody. As a result, since the amounts of the respective intrabodies after immunoprecipitation showed ratios equivalent to those of the cell lysates, it was determined that immunoprecipitation had been successfully performed.

The lower right panel of Fig. 1 is a graph showing the results of evaluating the amounts of KRAS bound to respective intrabodies, in which the luminance values of KRAS bound to the respective intrabodies after immunoprecipitation were logarithmically transformed and then averaged based on the results of Western blotting, and the amount of KRAS bound to Y13-259-HA without peptide attachment was used as a reference. The vertical axis of the graph indicates the amount (relative value) of KRAS bound to each intrabody. As a result, while KRAS could not be detected in Y13-259-HA without peptide attachment, the amount of KRAS was dramatically increased in Y13-259-HA with peptide3 composed of an acidic amino acid backbone attached. Even in Y13-259-HA with peptide8 composed of a neutral amino acid backbone attached, an increasing tendency in the amount of KRAS was observed, although the increase was smaller than that in Y13-259-HA with peptide3 attached. However, in Y 13-259-HA with peptide7 composed of a basic amino acid backbone attached, the increasing tendency in the amount of KRAS was found to be even smaller. These results suggest that the attached peptide improved the binding function of an antibody expressed within cells to a target antigen, and revealed that a peptide composed of an acidic amino acid backbone is important as a peptide for improving the binding function of an antibody to a target antigen within cells.

### [Experimental Example 2]

### (Investigation of peptide length capable of maintaining improvement in binding function of antibody to target antigen within cells)

Considering the use of intracellular antibodies for gene therapy applications, it is desirable for the peptide length to be as short as possible. Therefore, in order to confirm a range of peptide lengths composed of an acidic amino acid backbone capable of exerting a binding function of an antibody to a target antigen within cells, peptide1 (20 amino acids), peptide2 (15 amino acids), peptide3 (9 amino acids), peptide16 (3 amino acids), and peptide17 (6 amino acids), which differed in peptide length and were composed of an acidic amino acid backbone, were designed, and the effect of improving the binding function of an antibody to a target antigen within cells was evaluated by immunoprecipitation.

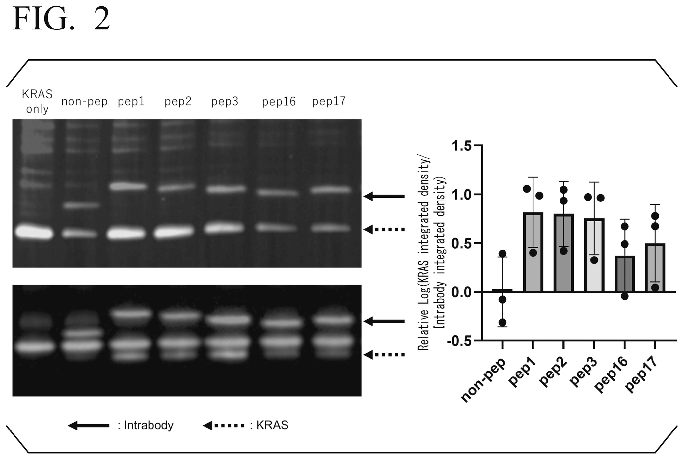

The upper left panel of Fig. 2 shows photographs of the results of Western blotting of the cell lysates (samples before immunoprecipitation). As a result, it was confirmed that each intrabody and KRAS were expressed in the cell lysates. The lower left panel of Fig. 2 shows photographs of the results of Western blotting of the samples after immunoprecipitation. In Fig. 2, "KRAS only" indicates the result obtained when only KRAS was expressed, "non-pep" indicates the result obtained when Y13-259-HA without peptide attachment was expressed, "pep1" indicates the result obtained when Y13-259-HA with peptide1 attached was expressed, and so forth.

The right panel of Fig. 2 is a graph showing the results of comparing the ratios of the amount of KRAS to the amount of each intrabody, in which the luminance values of each intrabody and KRAS contained in the samples after immunoprecipitation were calculated, the ratio of the amount of KRAS to the amount of intrabody (ratio = amount of KRAS / amount of intrabody) was logarithmically transformed and then averaged, and the ratio of the amount of KRAS to Y13-259-HA without peptide attachment was used as a reference. The vertical axis of the graph indicates the ratio (relative value) of the amount of KRAS to the amount of each intrabody.

As a result, Y13-259-HA with peptide1 to peptide3 attached was found to stably improve the binding function of an antibody to a target antigen within cells compared to Y13-259-HA without peptide attachment.

On the other hand, although Y13-259-HA with peptide16 or peptide17 attached also exhibited an effect of improving the binding function of an antibody to a target antigen within cells, it was shown that Y13-259-HA with the other peptides attached is more likely to exhibit this effect more stably. These results revealed that when the length of the acidic amino acid backbone peptide is 9 to 20 amino acids, the binding function of an antibody to a target antigen within cells can be improved particularly stably.

### [Experimental Example 3]

### (Investigation of content of other amino acids in acidic amino acid backbone)

It was shown in the investigation of Experimental Example 2 that even when a peptide having an acidic amino acid backbone contained 22.2% to 26.6% of proline (P), which is a neutral amino acid, as another type of amino acid, the peptide was able to maintain the function of improving the binding function of an antibody to a target antigen within cells. Furthermore, in order to determine a allowable range of the content of other amino acids in an acidic amino acid backbone, peptides (peptide12 to peptide15) containing 33.3%, 44.4%, 55.5%, or 66.6% of another amino acid (proline (P), which is a neutral amino acid, was used in this experiment) in peptides having an acidic amino acid backbone were designed, and the effect of improving the binding function of an antibody to a target antigen within cells was evaluated by immunoprecipitation.

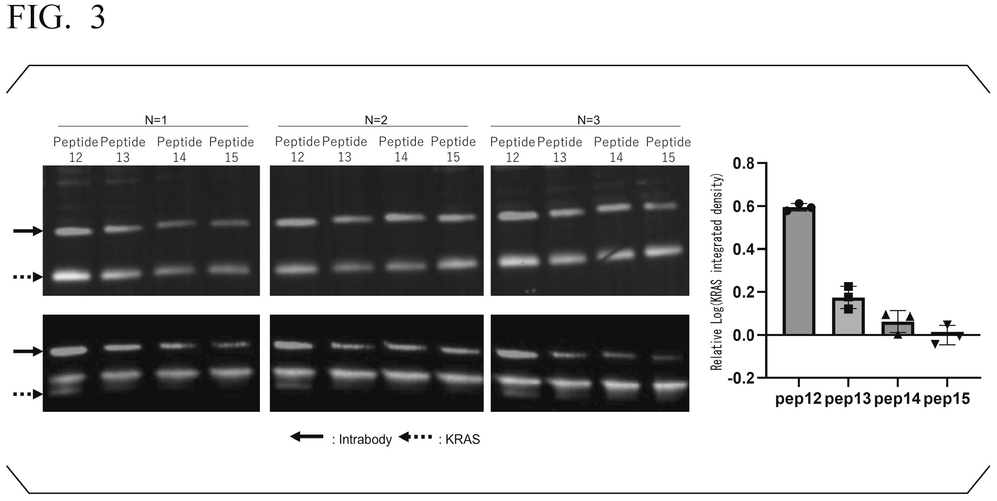

The upper left panel of Fig. 3 shows photographs of the results of Western blotting of the cell lysates (samples before immunoprecipitation). The lower left panel of Fig. 3 shows photographs of the results of Western blotting of the samples after immunoprecipitation. The right panel of Fig. 3 is a graph showing the results of comparing the amounts of KRAS bound to the respective intrabodies, in which the luminance values of KRAS bound to the respective intrabodies contained in the samples after immunoprecipitation were logarithmically transformed and then averaged, and the amount of KRAS bound to Y13-259-HA with peptide15 attached was used as a reference. In Fig. 3, "Peptide12" indicates the result obtained when Y13-259-HA with peptide12 attached was expressed, and so forth. The vertical axis of the graph indicates the amount (relative value) of KRAS bound to each intrabody.

As a result, in the cell lysates, substantially comparable levels of intrabodies and KRAS were expressed for all peptides. However, in the samples after immunoprecipitation, relatively stable binding to KRAS was observed for Y13-259-HA with peptide12 attached, whereas stable binding to KRAS could not be confirmed for the other peptides.

These results revealed that when the content of other amino acids in an acidic amino acid backbone is 33.3% or less, the binding function of an antibody to a target antigen within cells can be particularly stably improved.

### [Experimental Example 4]

### (Investigation of types of neutral amino acids in acidic amino acid backbones)

Regarding the design of polypeptides that improve the binding function of an antigen-binding polypeptide to a target antigen within cells, it was shown in Experimental Example 3 that proline (a hydrophobic/imino acid), which is one type of neutral amino acid, is permissible as a type of amino acid that can be introduced into an acidic amino acid backbone. Furthermore, in order to verify whether other types of neutral amino acids that can be introduced into an acidic amino acid backbone exist, glycine (G), classified as a hydrophobic/aliphatic amino acid, phenylalanine (F), classified as a hydrophobic/aromatic amino acid, and serine (S), classified as a hydrophilic amino acid, were selected as representative amino acids of respective properties, and peptides (peptide9 to peptide11) were designed in which proline in peptide3 was replaced with each of these amino acids, and the effect of improving the binding function of antibodies to target antigens within cells was evaluated by immunoprecipitation.

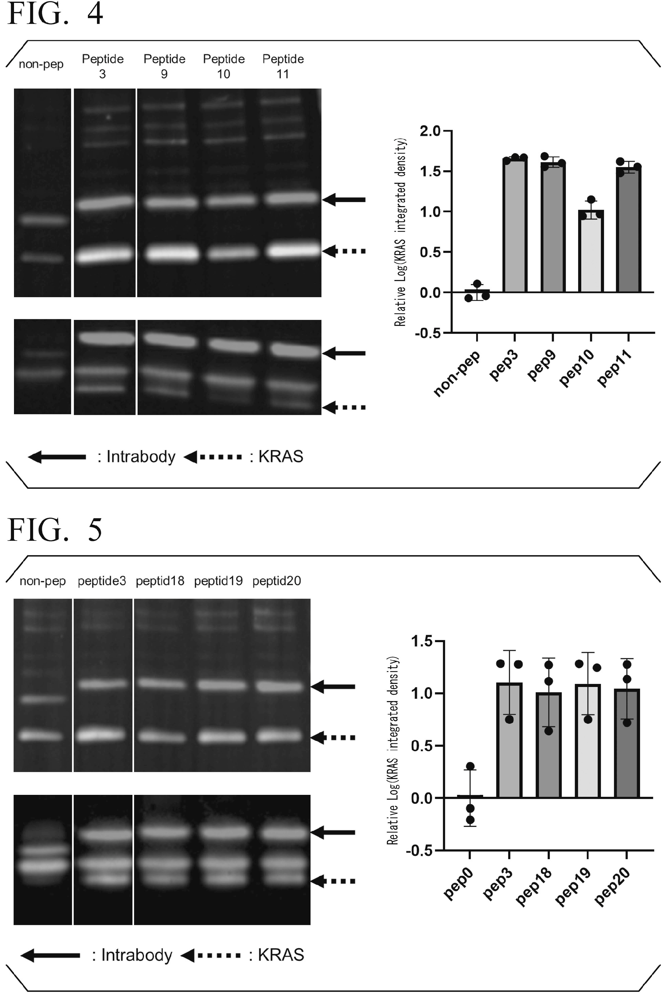

The upper left panel of Fig. 4 shows photographs of the results of Western blotting of the cell lysates (samples before immunoprecipitation). The lower left panel of Fig. 4 shows photographs of the results of Western blotting of the samples after immunoprecipitation. In Fig. 4, "non-pep" indicates the result obtained when Y13-259-HA without peptide attachment was expressed, "Peptide3" indicates the result obtained when Y13-259-HA with peptide3 attached was expressed, and so forth. As a result, it was confirmed that each intrabody and KRAS were expressed in the cell lysates in all samples.

The right panel of Fig. 4 is a graph showing the results of comparative evaluation of the amounts of KRAS bound to the respective intrabodies, in which the luminance values of KRAS bound to the respective intrabodies contained in the samples after immunoprecipitation were logarithmically transformed and averaged, and the amount of KRAS bound to Y13-259-HA without peptide attachment was used as a reference. The vertical axis of the graph indicates the amount (relative value) of KRAS bound to each intrabody.

As a result, after immunoprecipitation, an increasing tendency in the amount of binding to KRAS was confirmed for Y13-259-HA with peptide9 attached and Y13-259-HA with peptide11 attached. Even in Y13-259-HA with peptide10 attached, a slight increasing tendency in the amount of binding to KRAS was observed; however, the degree of increase was lower than that in Y13-259-HA with peptide9 attached and Y13-259-HA with peptide11 attached.

Based on these results, it is considered that, while amino acids other than proline are permissible as neutral amino acids that can be introduced into an acidic amino acid backbone in polypeptides that improve the binding function of an antigen-binding polypeptide to a target antigen within cells, neutral amino acids classified as hydrophobic/aliphatic or hydrophilic exhibit particularly favorable effects.

### [Experimental Example 5]

### (Investigation of types of basic amino acids in acidic amino acid backbones)

Regarding the design of polypeptides that improve the binding function of an antigen-binding polypeptide to a target antigen within cells, it was shown in Experimental Example 4 that introduction of a neutral amino acid into an acidic amino acid backbone is permissible. In Experimental Example 1, a basic amino acid backbone greatly reduced the effect of improving the binding function of an antibody to a target antigen within cells; however, in order to verify whether introduction into an acidic amino acid backbone is permissible, peptide18 to peptide20 were designed by replacing proline in peptide3 with lysine (K), and the effect of improving the target-binding function within cells was evaluated by immunoprecipitation.

The upper left panel of Fig. 5 shows photographs of the results of Western blotting of the cell lysates (samples before immunoprecipitation). The lower left panel of Fig. 5 shows photographs of the results of Western blotting of the samples after immunoprecipitation. In Fig. 5, "non-pep" indicates the result obtained when Y13-259-HA without peptide attachment was expressed, "Peptide3" indicates the result obtained when Y13-259-HA with peptide3 attached was expressed, and so forth. As a result, it was confirmed that each intrabody and KRAS were expressed in the cell lysates in all samples.

The right panel of Fig. 5 is a graph showing the results of comparative evaluation of the amounts of KRAS bound to the respective intrabodies, in which the luminance values of KRAS bound to the respective intrabodies contained in the samples after immunoprecipitation were logarithmically transformed and averaged, and the amount of KRAS bound to Y13-259-HA without peptide attachment (indicated as "pep0" in Fig. 5) was used as a reference. The vertical axis of the graph indicates the amount (relative value) of KRAS bound to each intrabody. As a result, Y13-259-HA with peptide1 8 to peptide20 attached was confirmed to bind to KRAS at levels comparable to that of peptide3.

These results revealed that introduction of a basic amino acid into an acidic amino acid backbone is permissible in polypeptides for improving the binding function of an antigen-binding polypeptide to a target antigen within cells.

### [Experimental Example 6]

### (Comparative investigation of effect of improving binding function of antibody to target antigen within cells)

Comparative evaluation by immunoprecipitation and Western blotting was performed on intracellular expression and binding function to the target antigen (KRAS-His) of Y13-259-HA with peptide4 (SEQ ID NO: 26, s3Flag), peptide5 (SEQ ID NO: 27, DE2.0), or peptide6 (SEQ ID NO: 28, DE5.0) attached thereto, each of which has been reported to improve aggregation properties of antibodies expressed within cells (see Patent Document 1 (WO 2019/004213) for all of these), and Y13-259-HA with peptide2 or peptide3, which were found in the present study to improve the binding function of antibodies to target antigens within cells, attached to the N-terminus thereof.

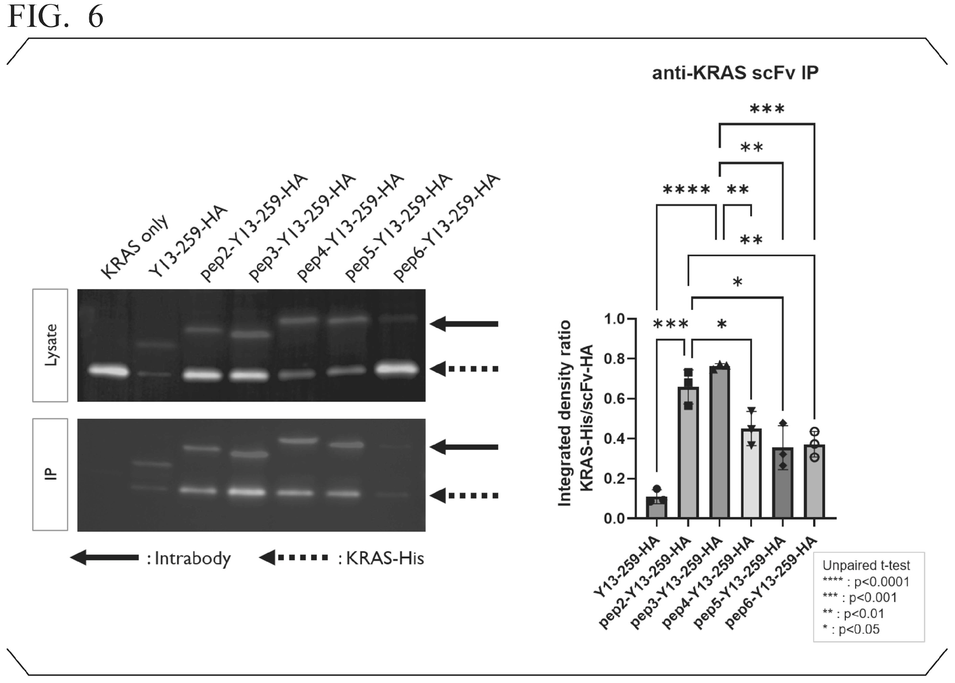

The upper left panel of Fig. 6 shows photographs of the results of Western blotting of the cell lysates (samples before immunoprecipitation). The lower left panel of Fig. 6 shows photographs of the results of Western blotting of the samples after immunoprecipitation. In Fig. 6, "KRAS only" indicates the result obtained when only KRAS-His was expressed, "Y13-259-HA" indicates the result obtained when Y13-259-HA without peptide attachment was expressed, "pep2-Y13-259-HA" indicates the result obtained when Y13-259-HA with peptide2 attached was expressed, and so forth. As a result, a significant decrease in expression level was observed only for Y13-259-HA with peptide6 attached. For the other samples, co-expression of each intrabody and KRAS was confirmed.

The right panel of Fig. 6 is a graph showing the results of comparing the ratios of the amount of KRAS-His to the amount of each intrabody (ratio = amount of KRAS-His / amount of intrabody), in which the luminance values of each intrabody and KRAS-His contained in the samples after immunoprecipitation were calculated. The vertical axis of the graph indicates the ratio of the amount of KRAS-His to the amount of each intrabody. As a result, it was confirmed that Y13-259-HA with peptide2, peptide3, peptide4, peptide5, or peptide6 attached exhibited improved binding function to KRAS-His compared to Y13-259-HA without peptide attachment. Furthermore, it was confirmed that Y13-259-HA with peptide2 or peptide3 attached exhibited significantly improved binding function to KRAS-His within cells compared to Y13-259-HA with peptide4, peptide5, or peptide6 attached.

These results confirmed that the polypeptide of the present embodiment, as compared to conventional polypeptides reported to improve the aggregation properties of antibodies expressed within cells, stably maintains the expression levels of antibodies and the like within cells and remarkably improve the binding function of antigen-binding polypeptides to target antigens.

### [Experimental Example 7]

### (Investigation of improvement in binding function of antigen-binding polypeptide to endogenous intracellular target antigen)

In the studies conducted thus far, KRAS-His overexpressed within cells has been used as the target antigen. In order to confirm whether the effect of improving the binding function exhibited by the polypeptide found in the present study is also observed for endogenous intracellular target antigens, the effect of improving the binding function to endogenous KRAS was evaluated by immunoprecipitation.

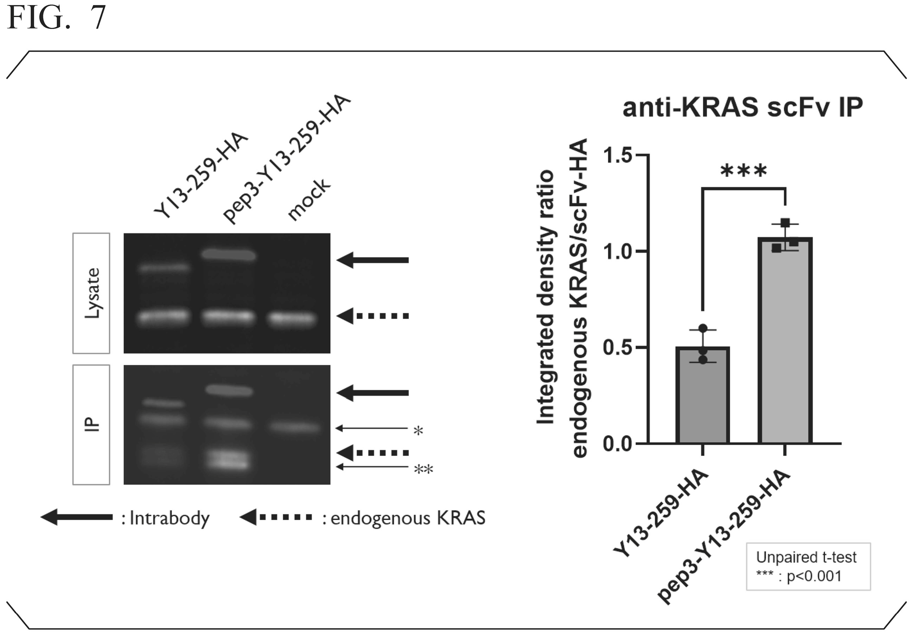

The upper left panel of Fig. 7 shows photographs of the results of Western blotting of the cell lysates (samples before immunoprecipitation). The lower left panel of Fig. 7 shows photographs of the results of Western blotting of the samples after immunoprecipitation. In Fig. 7, "Y13-259-HA" indicates the result obtained when Y13-259-HA without peptide attachment was expressed, "pep3-Y13-259-HA" indicates the result obtained when Y13-259-HA with peptide3 attached was expressed, and "mock" indicates the result obtained when an empty pcDNA3.4 vector was transfected as a negative control. As a result, it was confirmed that each intrabody was expressed in the cell lysates in Y13-259-HA and pep3-Y13-259-HA, and that endogenous intracellular KRAS was present. The band indicated by the arrow marked with "*" in the lower left panel of Fig. 7 was considered to be a nonspecific band derived from the beads used for immunoprecipitation. Further, the band indicated by the arrow marked with "**" was considered to be possibly a degradation product of KRAS.

The right panel of Fig. 7 is a graph showing the results of comparing the ratios of the amount of endogenous KRAS to the amount of each intrabody (ratio = amount of endogenous KRAS / amount of intrabody), in which the luminance values of endogenous KRAS bound to the respective intrabodies contained in the samples after immunoprecipitation were calculated. The vertical axis of the graph indicates the ratio of the amount of endogenous KRAS to the amount of each intrabody. As a result, it was confirmed that Y13-259-HA with peptide3 attached exhibited significantly improved binding function to endogenous intracellular KRAS compared to Y13-259-HA without peptide attachment.

From this result, it was found that the effect of the polypeptide of the present embodiment in improving the binding function is also effective for binding to endogenous intracellular target antigens.

### [Experimental Example 8]

### (Investigation of improvement in binding function of antigen-binding polypeptide to different target antigen)

In the studies conducted thus far, KRAS has been used as the target antigen. In order to confirm whether the effect of improving the binding function exhibited by the polypeptide found in the present study is also observed for other target antigens, the effect of improving the binding function of anti-β-galactosidase antibody scFv13 to β-galactosidase was evaluated by immunoprecipitation.

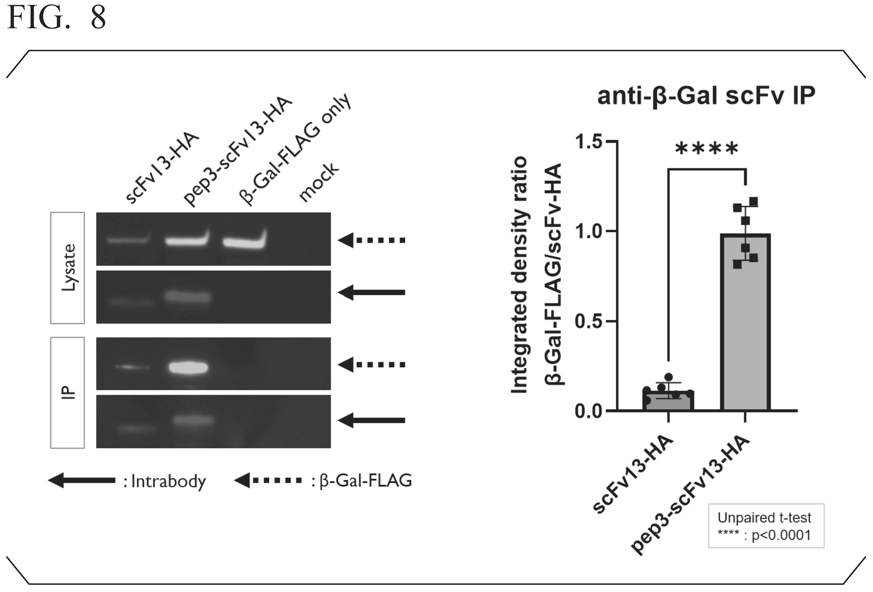

The upper left panel of Fig. 8 shows photographs of the results of Western blotting of the cell lysates (samples before immunoprecipitation). The lower left panel of Fig. 8 shows photographs of the results of Western blotting of the samples after immunoprecipitation. In Fig. 8, "scFv13-HA" indicates the result obtained when scFv13-HA without peptide attachment was expressed, "pep3-scFv13-HA" indicates the result obtained when scFv13-HA with peptide3 attached was expressed, "β-Gal-FLAG only" indicates the result obtained when only β-Gal-FLAG was expressed as a negative control, and "mock" indicates the result obtained when an empty pcDNA3.4 vector was transfected as a negative control. As a result, it was confirmed that each intrabody and β-Gal-FLAG were expressed in the cell lysates in scFv13-HA and pep3-scFv13-HA.

The right panel of Fig. 8 is a graph showing the results of comparing the ratio of the amount of β-Gal-FLAG to the amount of each intrabody (ratio = amount of β-Gal-FLAG / amount of intrabody), in which the luminance values of β-Gal-FLAG bound to the respective intrabodies contained in the samples after immunoprecipitation were calculated. The vertical axis of the graph indicates the ratio of the amount of β-Gal-FLAG to the amount of each intrabody. As a result, it was confirmed that scFv13-HA with peptide3 attached exhibited significantly improved binding function to β-Gal-FLAG compared to scFv13-HA without peptide attachment.

From this result, it was found that the effect of the polypeptide of the present embodiment in improving the binding function is also effective for binding to target antigens other than KRAS.

### [Experimental Example 9]

### (Investigation of improvement in binding function of antigen-binding polypeptide in different format (VH) to different target antigen)

In the studies conducted thus far, scFv has been used as the antigen-binding polypeptide. In order to confirm whether the effect of improving the binding function exhibited by the polypeptide found in the present study is also observed for antigen-binding polypeptides in other formats, the effect of improving the binding function of iDab#6, which is an anti-HRAS (G12V mutant) antibody VH, was evaluated by immunoprecipitation.

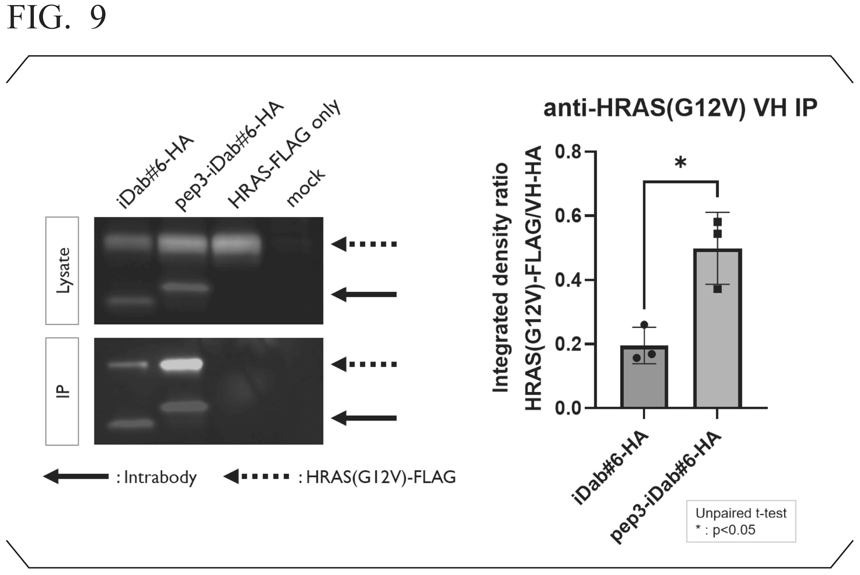

The upper left panel of Fig. 9 shows photographs of the results of Western blotting of the cell lysates (samples before immunoprecipitation). The lower left panel of Fig. 9 shows photographs of the results of Western blotting of the samples after immunoprecipitation. In Fig. 9, "iDab#6-HA" indicates the result obtained when iDab#6-HA without peptide attachment was expressed, "pep3-iDab#6-HA" indicates the result obtained when iDab#6-HA with peptide3 attached was expressed, "HRAS-FLAG only" indicates the result obtained when only HRAS (G12V)-FLAG was expressed as a negative control, and "mock" indicates the result obtained when an empty pcDNA3.4 vector was transfected as a negative control. As a result, it was confirmed that each intrabody and HRAS (G12V)-FLAG were expressed in the cell lysates in iDab#6-HA and pep3-iDab#6-HA.

The right panel of Fig. 9 is a graph showing the results of comparing the ratio of the amount of HRAS (G12V)-FLAG to the amount of each intrabody (ratio = amount of HRAS (G12V)-FLAG / amount of intrabody), in which the luminance values of HRAS (G12V)-FLAG bound to the respective intrabodies contained in the samples after immunoprecipitation were calculated. The vertical axis of the graph indicates the ratio of the amount of HRAS (G12V)-FLAG to the amount of each intrabody. As a result, it was confirmed that iDab#6-HA with peptide3 attached exhibited significantly improved binding function to HRAS (G12V)-FLAG compared to iDab#6-HA without peptide attachment.

From this result, it was found that the effect of improving the binding function of the polypeptide of the present embodiment is effective not only for antigen-binding polypeptides in the scFv format but also for those in the VH format.

### [Experimental Example 10]

### (Investigation of improvement in binding function of antigen-binding polypeptide in different format (shark antibody vNAR) to different target antigen)

Following the VH format study in Experimental Example 10, in order to confirm whether an effect is also observed in an antigen-binding polypeptide in the shark antibody vNAR format, the effect of improving the binding function of BsG73, which is an anti-EGFP antibody vNAR, was evaluated by immunoprecipitation.

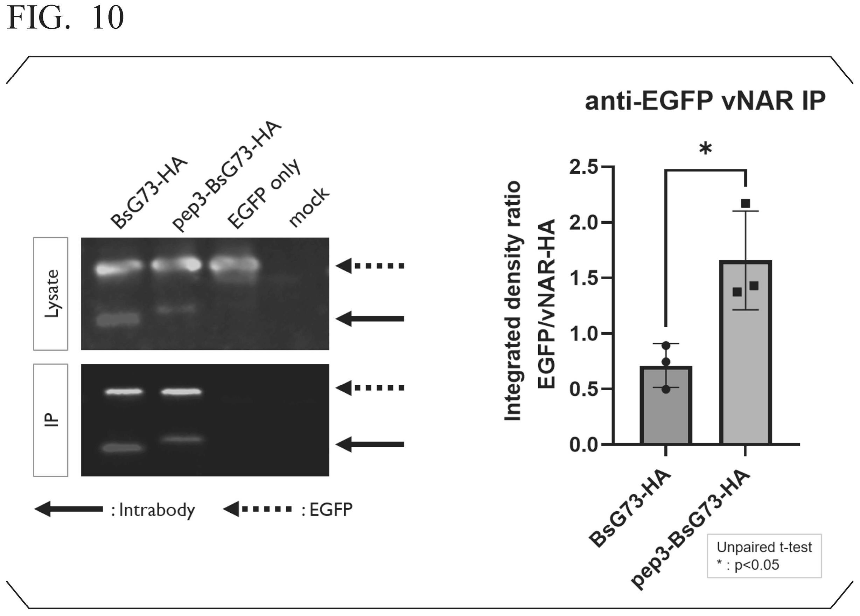

The upper left panel of Fig. 10 shows photographs of the results of Western blotting of the cell lysates (samples before immunoprecipitation). The lower left panel of Fig. 10 shows photographs of the results of Western blotting of the samples after immunoprecipitation. In Fig. 10, "BsG73-HA" indicates the result obtained when BsG73-HA without peptide attachment was expressed, "pep3-BsG73-HA" indicates the result obtained when BsG73-HA with peptide3 attached was expressed, "EGFP only" indicates the result obtained when only EGFP was expressed as a negative control, and "mock" indicates the result obtained when an empty pcDNA3.4 vector was transfected as a negative control. As a result, it was confirmed that each intrabody and EGFP in the cell lysates were expressed in BsG73-HA and pep3-BsG73-HA.

The right panel of Fig. 10 is a graph showing the results of comparing the ratios of the amount of EGFP to the amount of each intrabody (ratio = amount of EGFP / amount of intrabody), in which the luminance values of each intrabody and EGFP contained in the samples after immunoprecipitation were calculated. The vertical axis of the graph indicates the ratio of the amount of EGFP to the amount of each intrabody. As a result, it was confirmed that BsG73-HA with peptide3 attached exhibited significantly improved binding function to EGFP compared to BsG73-HA without peptide attachment.

From this result, it was found that the effect of improving the binding function of the polypeptide of the present embodiment is also effective for antigen-binding polypeptides in the shark antibody vNAR format.

### [Experimental Example 11]

### (Investigation of improvement in binding function of antigen-binding polypeptide in different format (camelid antibody VHH) to different target antigen)

In order to confirm whether an effect is also observed in an antigen-binding polypeptide in the camelid antibody VHH format, the effect of improving the binding function of "enhancer," which is an anti-EGFP antibody VHH, was evaluated by immunoprecipitation.

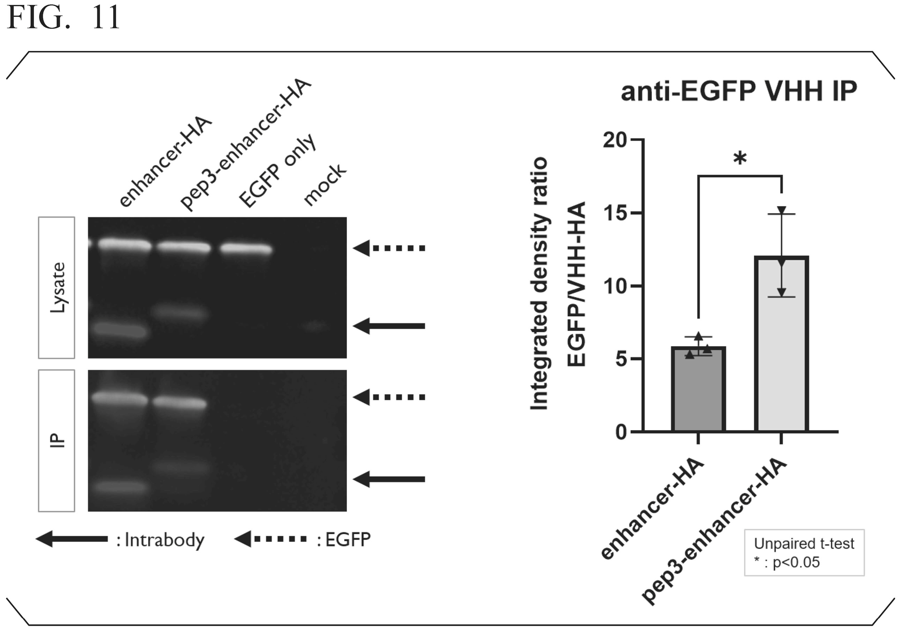

The upper left panel of Fig. 11 shows photographs of the results of Western blotting of the cell lysates (samples before immunoprecipitation). The lower left panel of Fig. 11 shows photographs of the results of Western blotting of the samples after immunoprecipitation. In Fig. 11, "enhancer-HA" indicates the result obtained when enhancer-HA without peptide attachment was expressed, "pep3-enhancer-HA" indicates the result obtained when enhancer-HA with peptide3 attached was expressed, "EGFP only" indicates the result obtained when only EGFP was expressed as a negative control, and "mock" indicates the result obtained when an empty pcDNA3.4 vector was transfected as a negative control. As a result, it was confirmed that each intrabody and EGFP in the cell lysates were expressed in enhancer-HA and pep3-enhancer-HA.

The right panel of Fig. 11 is a graph showing the results of comparing the ratios of the amount of EGFP to the amount of each intrabody (ratio = amount of EGFP / amount of intrabody), calculated from the luminance values of the respective intrabodies and EGFP bound thereto in the samples after immunoprecipitation. The vertical axis of the graph indicates the ratio of the amount of EGFP to the amount of each intrabody. As a result, it was confirmed that enhancer-HA with peptide3 attached exhibited significantly improved binding function to EGFP compared to enhancer-HA without peptide attachment.

From this result, it was found that the effect of improving the binding function of the polypeptide of the present embodiment is also effective for antigen-binding polypeptides in the camelid antibody VHH format.

From the results obtained thus far, it was found that the effect of improving the binding function of the polypeptide of the present embodiment is effective for antigen-binding polypeptides of all antibody formats and for all target antigens.

### [Experimental Example 12]

### (Comparative investigation of effect of reducing suppression of cell proliferation)

In addition to Experimental Example 6, comparative evaluation was performed on the effects on cell proliferation when expressed in 293A cells, for Y13-259-HA with peptide4 (SEQ ID NO: 26, s3Flag), peptide5 (SEQ ID NO: 27, DE2.0), or peptide6 (SEQ ID NO: 28, DE5.0) attached thereto, each of which has been reported to improve aggregation properties of antibodies expressed within cells (see Patent Document 1 (WO 2019/004213) for all of these), and Y13-259-HA with peptide2 or peptide3, which were found in the present study to improve the binding function of antibodies to target antigens within cells, attached to the N-terminus thereof. After transfection of each vector, the cells were imaged over time, and the confluence (%) of the 293A cells was calculated based on the obtained images.

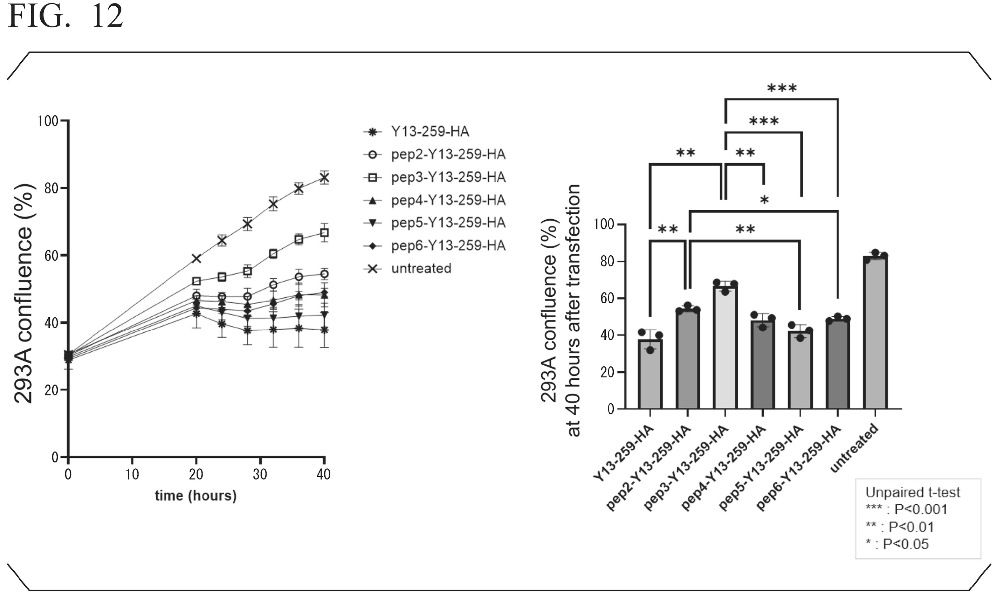

The left panel of Fig. 12 is a graph showing cell proliferation after transfection over time using confluence (%) as an indicator. The vertical axis of the graph indicates the confluence (%) of the 293A cells, and the horizontal axis indicates the elapsed time after transfection. The right panel of Fig. 12 is a bar graph in which the vertical axis indicates the confluence (%) at 40 hours after transfection. In Fig. 12, "Y13-259-HA" indicates the result obtained when Y13-259-HA without peptide attachment was expressed, "pep2-Y13-259-HA" indicates the result obtained when Y13-259-HA with peptide2 attached was expressed, and so forth. Further, "untreated" indicates the result obtained without performing transfection.

As a result, it was confirmed that cell proliferation was suppressed in cells expressing Y13-259-HA compared to untreated cells. Furthermore, it was confirmed that when Y13-259-HA with peptide2, peptide3, peptide4, or peptide6 attached was expressed, the suppression of cell proliferation was significantly reduced.

In addition, it was confirmed that Y13-259-HA with peptide3 attached exhibited significantly improved effect of reducing the suppression of cell proliferation compared to Y13-259-HA with peptide4, peptide5, or peptide6 attached.

It was confirmed that Y13-259-HA with peptide2 attached exhibited a significantly improved effect of reducing the suppression of cell proliferation compared to Y13-259-HA with peptide5 or peptide6 attached. Further, a tendency to further reduce the suppression of cell proliferation was observed even when compared to Y13-259-HA with peptide4 attached.

From the above results, it was confirmed that the polypeptide of the present embodiment significantly reduces the suppression of cell proliferation compared to conventional polypeptides that have been reported to have an effect of improving the aggregation properties of antibodies expressed within cells.

### Industrial Applicability

According to the polypeptide of the present embodiment, binding of an antigen-binding polypeptide to an antigen within cells can be improved.

## Claims

1. A polypeptide for improving binding of an antigen-binding polypeptide to an antigen within cells, the polypeptide comprising 3 or more and 20 or less amino acids, wherein more than 57.0% and less than 100.0% of the amino acids are acidic amino acids.

2. The polypeptide according to claim 1, comprising 9 or more and 20 or less amino acids.

3. The polypeptide according to claim 1, wherein 60.0% or more and 90.0% or less of the amino acids are acidic amino acids.

4. The polypeptide according to claim 3, wherein 65.0% or more and 80.0% or less of the amino acids are acidic amino acids.

5. The polypeptide according to claim 1, having a glutamic acid at its N-terminus.

6. The polypeptide according to claim 1, comprising acidic amino acids and neutral amino acids.

7. The polypeptide according to claim 6, wherein 10.0% or more and 40.0% or less of the amino acids are neutral amino acids.

8. The polypeptide according to claim 7, wherein 20.0% or more and 35.0% or less of the amino acids are neutral amino acids.

9. The polypeptide according to claim 6, wherein side chains of the neutral amino acids are each a hydrophobic aliphatic group or a hydrophilic group.

10. The polypeptide according to claim 9, wherein the neutral amino acids are proline, glycine, or serine.

11. The polypeptide according to claim 1, further comprising basic amino acids, wherein the basic amino acids are lysine.

12. The polypeptide according to claim 1, comprising an amino acid sequence represented by the following general formula (I) or (II):
[Chemical Formula 1] (EX¹D)ₙ₁EX²X³(EX⁴D)ₙ₂ (I)
in general formula (1),
X¹ and X⁴ are each independently a neutral amino acid or a basic amino acid;
X² is D or a neutral amino acid;
X³ is D or E; and
n1 and n2 are each independently an integer of 0 or more and 3 or less, and 0 ≦ n1 + n2 ≦ 5, and
[Chemical Formula 2] (EX¹DEX²X³EX⁴D)ₙ₃ EX⁵(EX¹DEX²X³EX⁴D)ₙ₄ (II)
in general formula (II),
X¹ and X⁴ are each independently a neutral amino acid or a basic amino acid;
X² is D or a neutral amino acid;
X³ is D or E;
X⁵ is a neutral amino acid or a basic amino acid; and
n3 and n4 are each independently an integer of 0 or more and 1 or less, and satisfy a relational expression: 1 ≦ n3 + n4 ≦ 2.

13. The polypeptide according to claim 12, comprising an amino acid sequence represented by any one of SEQ ID NOs: 64 to 66, 72, and 82.

14. The polypeptide according to claim 1, comprising an amino acid sequence represented by any one of SEQ ID NOs: 23 to 25, 31, 33, 34, and 40 to 42.

15. A fusion polypeptide comprising the polypeptide according to any one of claims 1 to 14 and an antigen-binding polypeptide.

16. The fusion polypeptide according to claim 15, wherein the antigen-binding polypeptide includes one or more complementarity-determining regions of an antibody against a specific antigen.

17. A nucleic acid encoding the polypeptide according to any one of claims 1 to 14.

18. The nucleic acid according to claim 17, comprising a nucleotide sequence represented by any one of SEQ ID NOs: 3 to 5, 11, 13, 14, 19, and 20 to 22.

19. An expression vector comprising the nucleic acid according to claim 17.

20. The expression vector according to claim 19, wherein the nucleic acid includes a nucleotide sequence represented by any one of SEQ ID NOs: 3 to 5, 11, 13, 14, 19, and 20 to 22.

21. A nucleic acid encoding the fusion polypeptide according to claim 15.

22. An expression vector comprising the nucleic acid according to claim 21.
